# EUROPÄISCHE PATENTSCHRIFT

(11) **EP 2 467 717 B1**
(45) Veröffentlichungstag und Bekanntmachung des Hinweises auf die Patenterteilung: **31.12.2014**
(21) Anmeldenummer: 10743187.6
(22) Anmeldetag: 20.08.2010
(51) Int. Cl.: G01N 33/487

(54) **Vorratsbehälter mit diagnostischem Testelement**
Container with diagnostic test element
Récipient avec élément de test diagnostique

(30) Priorität: 20.08.2009 EP 09168331
(43) Veröffentlichungstag der Anmeldung: 27.06.2012
(60) Teilanmeldung: 14193721.9
(73) Patentinhaber: F.Hoffmann-La Roche AG, 4070 Basel (CH); Roche Diagnostics GmbH, 68305 Mannheim (DE)
(72) Erfinder: HORN, Carina, 68647 Biblis (DE); FREITAG, Christian, 55278 Weinolsheim (DE); HAAR, Hans-Peter, 69168 Wiesloch (DE); EIKMEIER, Heino, 64653 Lorsch (DE)
(74) Vertreter: Weiss, Wolfgang
(86) Internationale Anmeldenummer: PCT/EP2010/062179
(87) Internationale Veröffentlichungsnummer: WO 2011/020914

(56) Entgegenhaltungen:
- EP-A- 1 801 584
- EP-A1- 1 914 547
- WO-A-2006/002432
- WO-A-2007/012494
- WO-A2-2010/094427
- US-A1- 2004 258 564

## Beschreibung

Die vorliegende Erfindung betrifft Vorratsbehälter zur Lagerung mindestens eines diagnostischen Testelements, welches ein Enzym und ein stabilisiertes Coenzym umfasst, sowie analytische Messgeräte, welche derartige Vorratsbehälter oder diagnostische Produkte umfassen.

Diagnostische Testelemente sind wichtige Bestandteile klinisch relevanter Analyseverfahren. Hierbei steht die Messung von Analyten, z.B. Metaboliten oder Substraten, im Vordergrund, welche beispielsweise mit Hilfe eines für den Analyten spezifischen Enzyms direkt oder indirekt bestimmt werden. Die Analyten werden hierbei mit Hilfe eines Enzym-Coenzym-Komplexes umgesetzt und anschließend quantifiziert. Dabei wird der zu bestimmende Analyt mit einem geeigneten Enzym, einem Coenzym und gegenenfalls einem Mediator in Kontakt gebracht, wobei das Coenzym durch die enzymatische Reaktion physikochemisch verändert, z.B. oxidiert bzw. reduziert, wird. Sofern zusätzlich ein Mediator zum Einsatz gelangt, überträgt dieser die bei Umsetzung des Analyten freigesetzten Elektronen vom reduzierten Coenzym üblicherweise auf einen optischen Indikator oder die leitfähigen Bestandteile einer Elektrode, so dass der Vorgang beispielsweise photometrisch oder elektrochemisch erfasst werden kann. Eine Kalibrierung liefert einen direkten Zusammenhang des Messwerts mit der Konzentration des zu bestimmenden Analyten.

Ein wichtiges Kriterium bei der Bereitstellung diagnostischer Testelemente ist deren Langzeitstabilität. Aus dem Stand der Technik bekannte Testelemente, welche beispielsweise bei der Bestimmung von Blutglucose verwendet werden, weisen im Allgemeinen eine hohe Empfindlichkeit gegenüber Feuchtigkeit und Wärme auf, wobei üblicherweise speziell Coenzym und Mediator in ihrer Funktion beeinträchtigt werden. Bei bestimmten Anwendungsformen, z.B. bei Tests, die vom Endverbraucher selbst durchgeführt werden, können daher durch eine falsche, unbemerkte Fehllagerung des Messsystems Fehlergebnisse vorkommen, welche vom Verbraucher kaum zu erkennen sind und ggf. zu einer Fehlbehandlung der jeweiligen Erkrankung führen können.

Insofern sind bei der Herstellung bzw. Lagerung herkömmlicher diagnostischer Testelemente besondere Schutzmaßnahmen erforderlich, welche einen Kontakt der Testchemie des diagnostischen Testelements insbesondere mit Feuchtigkeit verhindern. Dies kann beispielsweise durch Einbringung von Trocknungsmitteln, wie z. B. Kieselgel oder Molekularsieb, oder Dichtungselementen in einen das Testelement enthaltenden Vorratsbehälter bewerkstelligt werden.

Folglich besitzen derartige Testelemente den Nachteil, dass sie nach Entnahme aus dem Vorratsbehälter innerhalb weniger Minuten verbraucht werden müssen, um eine einwandfreie Funktionsfähigkeit zu gewährleisten. Sofern das Testelement nach Entnahme aus dem Vorratsbehälter oder einer Primärverpackung in ein analytisches Messgerät eingesetzt werden soll, muss das Testelement ferner bis zu seinem vollständigen Verbrauch trocken gehalten werden, was einen hohen apparativen Aufwand erfordert und die Ausgestaltung sowohl des Testelements als auch des analytischen Messgeräts erheblich einschränkt.

Die der Erfindung zugrunde liegende Aufgabe bestand somit darin, einen Vorratsbehälter für diagnostische Testelemente bereitzustellen, bei welchem die Nachteile des Standes der Technik zumindest teilweise beseitigt sind. Insbesondere sollte das diagnostische Testelement in dem Vorratsbehälter ohne hohen apparativen Aufwand über einen längeren Zeitraum in Gegenwart von Feuchtigkeit oder/und Wärme gelagert werden können, ohne hierbei einen merklichen Verlust an Enzymaktivität zu erleiden.

Diese Aufgabe wurde erfindungsgemäß gelöst mittels eines Vorratsbehälters, enthaltend mindestens ein diagnostisches Testelement, welches ein Enzym und ein stabilisiertes Coenzym umfasst, wobei der Vorratsbehälter im wesentlichen frei von Trocknungsmitteln ist und keine Dichtungselemente umfasst, welche das Eindringen von Feuchtigkeit aus der Umgebung in den Vorratsbehälter im wesentlichen verhindern.

In einer zweiten Ausführungsform wird obige Aufgabe gelöst mittels eines Vorratsbehälters, enthaltend mindestens ein diagnostisches Testelement, welches ein Enzym und ein stabilisiertes Coenzym umfasst, wobei der Vorratsbehälter einen Feuchtigkeitsaustausch zwischen unverbrauchten Testbereichen und verbrauchtem Testbereichen des Testelements oder der Testelemente ermöglicht.

Überraschenderweise wurde im Rahmen der vorliegenden Erfindung festgestellt, dass diagnostische Testelemente, welche ein Enzym und ein stabilisiertes Coenzym in ihrer Chemiebeschichtung umfassen, selbst bei erhöhter relativer Luftfeuchtigkeit oder/und bei erhöhten Temperaturen über mehrere Wochen bzw. Monate keiner merklichen Abnahme der Enzymaktivität unterliegen, womit der apparative Aufwand zur Vermeidung des Eindringens von Feuchtigkeit in einen das Testelement enthaltenden Vorratsbehälter reduziert werden kann. Gleichzeitig wird hierdurch eine einfachere und sichere Handhabung des diagnostischen Testelements durch den Anwender gewährleistet.

Diese Erkenntnis ist überraschend, da bekannt ist, dass Enzyme in Gegenwart von nativem Coenzym zwar eine erhöhte Kurzzeitstabilität für einige Stunden besitzen (Bertoldi et al., Biochem. J. (2005), 389, 885; van den Heuvel et al., J. Biol. Chem. (2005), 280, 32115; und Pan et al., J. Chin. Biochem. Soc. (1974), 3, 1), jedoch eine geringere Haltbarkeit über einen längeren Zeitraum aufweisen (Nutrition Reviews (1978), 36, 251). Die nunmehr beobachtete Langzeitstabilität diagnostischer Testelemente, welche ein Enzym und ein stabilisiertes Coenzym umfassen, gegenüber Feuchtigkeit oder/und Wärme ist umso mehr überraschend, als stabilisierte Coenzyme eine niedrigere Bindungskonstante mit dem Enzym aufweisen als die korrespondierenden nativen Coenzyme.

Der Begriff "Vorratsbehälter", wie er im Rahmen der vorliegenden Anmeldung verwendet wird, bezeichnet einen Behälter, welcher in ein analytisches Messgerät eingesetzt werden kann und derart ausgebildet ist, dass jeweils ein einzelner Testbereich mindestens eines in dem Behälter gelagerten diagnostischen Testelements, welches einen einzigen Testbereich oder mehrere Testbereiche umfassen kann, mit dem analytischen Messgerät wechselwirken kann. Die Wechselwirkung zwischen dem Testbereich des diagnostischen Testelements und dem analytischen Messgerät kann grundsätzlich auf beliebige Art und Weise, insbesondere jedoch optisch, beispielsweise durch Bestrahlen des mit einer Probe des Analyten benetzten Testbereichs mit Licht geeigneter Wellenlänge und anschließendes Detektieren der von dem Testbereich emittierten Strahlung mittels des analytischen Messgeräts, erfolgen.

Um eine qualitative oder/und quantitative Bestimmung des Analyten mittels eines analytischen Messgeräts zu ermöglichen, umfassen die erfindungsgemäßen Vorratsbehälter vorzugsweise eine Probenaufgabeposition, in welcher ein Testbereich des in dem Vorratsbehälter gelagerten diagnostischen Testelements mit einer Probe des Analyten in Kontakt gebracht werden kann, eine Messposition, in welcher der Analyt durch Wechselwirkung von Testbereich des diagnostischen Testelements und analytischem Messgerät bestimmt werden kann, sowie mindestens eine Lagerungsposition, in welcher der Testbereich des diagnostischen Testelements vor oder/und nach Inkontaktbringen mit der Probe und anschließender Bestimmung des Analyten gelagert werden kann.

Die hierin beschriebenen Vorratsbehälter umfassen grundsätzlich jede dem Fachmann geläufige und für die Zwecke der vorliegenden Erfindung geeignet erscheinende physikalische Form, sofern sie die Aufnahme mindestens eines diagnostischen Testelements, insbesondere eines Bandmagazins oder Teststreifenmagazins, gestatten. Die Vorratsbehälter können beispielsweise rund, abgerundet oder eckig ausgestaltet sein, wobei durch Einsatz runder oder abgerundeter Vorratsbehälter eine besonders hohe Dichtigkeit erzielt werden kann. Da die erfindungsgemäß verwendeten diagnostischen Testelemente eine hohe Stabilität der Testchemie aufweisen, ist eine extrem hohe Dichtigkeit indessen nicht zwingend erforderlich, womit auch eine eckige Ausgestaltung der Vorratsbehälter gut realisiert werden kann.

Konkrete Beispiele für Vorratsbehälter im Sinne der vorliegenden Erfindung umfassen u.a. Beutel, Boxen, Dosen, Einfachspender sowie Tüten, welche vorzugsweise eine Vielzahl einzelner diagnostischer Testelemente, beispielsweise eine Vielzahl miteinander verbundener, einzeln abtrennbarer diagnostischer Testelemente, enthalten und ihrerseits, insbesondere zum Zwecke des Transports, zusammen mit weiteren erfindungsgemäßen Vorratsbehältern in einer geeigneten Umverpackung aufgenommen sein können. Der Begriff "Vielzahl", wie er in vorliegender Anmeldung verwendet wird, bezeichnet dabei jegliche Zahl > 1.

Um den Eintritt von Staubpartikeln oder anderem partikulärem, die Bestimmung des Analyten beeinträchtigendem Material aus der äußeren Umgebung in ihr Inneres zu verhindern, weisen die erfindungsgemäßen Vorratsbehälter in einer bevorzugten Variante ausschließlich Eintrittsöffnungen mit einem Durchmesser von ≤ 100 µm, insbesondere von ≤ 20 µm, auf. Der Begriff "Eintrittsöffnung", wie er in vorliegender Anmeldung verwendet wird, umfasst dabei jegliche Öffnung, durch welche Partikel eines anorganischen oder/und organischen Materials aus der äußeren Umgebung der Vorratsbehälter in deren Inneres eindringen können, wie beispielsweise die Poren des zur Herstellung der Vorratsbehälter verwendeten Materials.

Der erfindungsgemäße Vorratsbehälter kann aus jedem beliebigen Material bestehen, welches dem Fachmann für die Zwecke einer Lagerung diagnostischer Testelemente geeignet erscheint. Vorzugsweise besteht der Vorratsbehälter allerdings zumindest teilweise, d. h. teilweise oder vollständig, aus mindestens einem wasserdampfdurchlässigen Material, welches gegebenenfalls noch Zusatzstoffe, wie beispielsweise Farbstoffe, Glättungsstoffe, Alterungsinhibitoren oder/und UV-Inhibitoren, umfassen kann. Als wasserdampfdurchlässiges Material kann erfindungsgemäß beispielsweise Esspapier, beschichtete Folien, Holz, Kunststoff, Papier, Pappe, Pergament, Pressspan oder Sperrholz eingesetzt werden, wobei Kunststoff als bevorzugt gilt. Besonders bevorzugt gelangt im Rahmen der vorliegenden Erfindung ein Vorratsbehälter zur Anwendung, welcher als wasserdampfdurchlässiges Material einen Kunststoff ausgewählt aus der Gruppe bestehend aus Polyamid, Polycarbonat, Polyester, Polyethylen und Polypropylen umfasst.

Wird als Vorratsbehälter ein Bandmagazin verwendet, so umfasst dieses bevorzugt ein einziges diagnostisches Testelement, wobei das diagnostische Testelement seinerseits üblicherweise mehr als einen Testbereich, bevorzugt mindestens 10, stärker bevorzugt mindestens 25, und am stärksten bevorzugt mindestens 50 einzelne Testbereiche umfasst. Findet als Vorratsbehälter ein Teststreifenmagazin Anwendung, so umfasst dieses bevorzugt mindestens 3, stärker bevorzugt mindestens 5, am stärksten bevorzugt mindestens 7 diagnostische Testelemente, wobei die diagnostischen Testelemente in diesem Fall üblicherweise nur einen einzigen Testbereich umfassen, bei entsprechendem Bedarf jedoch mehr als einen Testbereich aufweisen können.

Vorratsbehälter, welche für die Zwecke der vorliegenden Erfindung von Nutzen sind, können einen oder mehrere räumlich voneinander getrennte Bereiche zur Lagerung des mindestens einen diagnostischen Testelements umfassen. Sofern das diagnostische Testelement mehr als einen Testbereich umfasst, welcher mit einer den Analyten enthaltenden Probe in Kontakt gebracht werden kann, so umfasst der erfindungsgemäße Vorratsbehälter vorzugsweise einen oder zwei räumlich voneinander getrennte Bereiche, wie beispielsweise eine oder zwei Kammern, in welchen die Testbereiche des diagnostischen Testelements gelagert werden können.

Umfasst der Vorratsbehälter zwei räumlich voneinander getrennte Bereiche zur Lagerung des diagnostischen Testelements, so kann ein Bereich beispielsweise zur Lagerung unverbrauchter Testbereiche eines diagnostischen Testelements verwendet werden, während der zweite Bereich zur Lagerung verbrauchter Testbereiche des diagnostischen Testelements vorgesehen ist. Auf diese Weise kann sichergestellt werden, dass unverbrauchte Testbereiche und verbrauchte Testbereiche des diagnostischen Testelements innerhalb des Vorratsbehälters nicht direkt miteinander in Kontakt treten können. Es kann auch jedes diagnostische Testelement vor oder nach Benutzung in einem eigenen Bereich, wie beispielsweise in einer eigenen Kammer, gelagert werden.

Im Falle von Vorratsbehältern, welche lediglich einen Bereich, wie beispielsweise eine einzelne Kammer, zur Lagerung eines diagnostischen Testelements mit mehr als einem Testbereich umfassen, sind sowohl unverbrauchte Testbereiche als auch verbrauchte Testbereiche des diagnostischen Testelements in dem einen Bereich gelagert. Durch den Verzicht auf eine zweite Kammer bzw. physikalische Mittel wie beispielsweise eine Trennwand, welche einen Kontakt zwischen unverbrauchten Testbereichen und verbrauchten Testbereichen innerhalb des Vorratsbehälters verhindern, kann sowohl die Herstellung der Vorratsbehälter als auch die Lagerung der Testelemente erheblich vereinfacht und damit wirtschaftlicher gestaltet werden. Der Ausdruck "unverbrauchter Testbereich", wie er in vorliegender Anmeldung verwendet wird, bezeichnet dabei einen Testbereich eines in vorliegender Anmeldung beschriebenen diagnostischen Testelements vor Inkontaktbringen des Testbereichs mit einer Probe eines zu bestimmenden Analyten; der Ausdruck "verbrauchter Testbereich" wird demgegenüber zur Bezeichnung eines Testbereichs nach Inkontaktbringen des Testbereichs mit einer Probe eines zu bestimmenden Analyten verwendet.

In einer bevorzugten Ausführungsform handelt es sich bei den erfindungsgemäßen Vorratsbehältern um Bandmagazine oder Teststreifenmagazine, wobei Teststreifenmagazine als bevorzugt gelten. Bandmagazine, welche zwei Kammern zur getrennten Lagerung unverbrauchter Testbereiche und verbrauchter Testbereiche eines in Form eines Testbandes vorliegenden diagnostischen Testelements aufweisen, sind beispielsweise in DE 10 2005 013 685 A1, EP 1 739 432 A1 und WO 2004/047642 A1 beschrieben. Teststreifenmagazine im Sinne der vorliegenden Anmeldung sind dem Fachmann allgemein bekannt und umfassen insbesondere Blistermagazine, Leporellomagazine, Scheibenmagazine, Stapelmagazine, Trommelmagazine und Wendemagazine, welche beispielsweise in EP 0 951 939 A2, EP 1 022 565 A2, EP 1 736 772 A1, WO 2005/104948 A1 und der Zwischenveröffentlichung WO 2010/094427 beschrieben sind.

In der ersten Ausführungsform ist der erfindungsgemäße Vorratsbehälter im wesentlichen frei von Trocknungsmitteln. Der Ausdruck "im wesentlichen frei von Trocknungsmitteln" bedeutet hierbei, dass der Vorratsbehälter eine Menge von < 5 Gew.%, bevorzugt eine Menge von < 1 Gew.%, stärker bevorzugt eine Menge von < 0.1 Gew.%, noch stärker bevorzugt eine Menge von < 0.01 Gew.%, und am stärksten bevorzugt eine Menge von < 0.001 Gew.% an Trocknungsmittel, bezogen auf das Leergewicht des Vorratsbehälters, enthält. Die Möglichkeit der signifikanten Reduzierung der Menge an Trocknungsmittel bzw. eines vollständigen Verzichts auf Trocknungsmittel im Rahmen der Herstellung und Lagerung der hierin beschriebenen diagnostischen Testelemente hat den Vorteil, dass sowohl Herstellung als auch Lagerung der Testelemente vereinfacht und damit wirtschaftlicher gestaltet werden kann.

Sofern der erfindungsgemäße Vorratsbehälter nicht vollständig frei von Trocknungsmittel ist, d. h. ein Trocknungsmittel in einer der oben angegebenen Mengen enthält, so kann das Trocknungsmittel separat in den Vorratsbehälter eingebracht oder/und in das Gehäuse des Vorratsbehälters integriert sein. Enthält der Vorratsbehälter das Trocknungsmittel in seinem Gehäuse integriert, so bedeutet dies, dass das Trocknungsmittel in das feste Gehäuse des Vorratsbehälters eingebracht ist und mindestens eine innere Oberfläche des Vorratsbehälters die Aufnahme von Feuchtigkeit aus dem Inneren des Vorratsbehälters ermöglicht. Derartige Vorratsbehälter können beispielsweise durch Einspritzen des Trocknungsmittels in eine zur Herstellung des Gehäuses verwendete Form und anschließendes Abkühlen/Aushärten der plastischen Zusammensetzung erzeugt werden.

In der ersten Ausführungsform enthält der erfindungsgemäße Vorratsbehälter keine Dichtungselemente, welche das Eindringen von Feuchtigkeit aus der Umgebung in den Vorratsbehälter im wesentlichen verhindern. Der Ausdruck "Dichtungselemente, welche das Eindringen von Feuchtigkeit aus der Umgebung in den Vorratsbehälter im wesentlichen verhindern" bedeutet, dass die Dichtungselemente das Eindringen von Feuchtigkeit aus der Umgebung in den Vorratsbehälter vollständig verhindern oder lediglich das Eindringen einer Menge an Feuchtigkeit gestatten, welche über einen Zeitraum von mindestens 4 Wochen, bevorzugt von mindestens 8 Wochen, und besonders bevorzugt von mindestens 12 Wochen zu keiner Beeinträchtigung der Testchemie des diagnostischen Testelements, beispielsweise durch Inaktivierung von Coenzym und gegebenenfalls Mediator, führt.

Beispiele für Dichtungselemente im Sinne der vorliegenden Anmeldung umfassen insbesondere Dichtungen und dichte Versiegelungen, sind jedoch nicht auf diese beschränkt. Dichtungselemente, welche das Eindringen von Feuchtigkeit aus der Umgebung in die hierin beschriebenen Vorratsbehälter gestatten und in einer bevorzugten Ausführungsform der Erfindung Anwendung finden können, dienen somit hauptsächlich dem Schutz der in dem Vorratsbehälter gelagerten diagnostischen Testelemente vor Staubpartikeln bzw. mechanischen Beschädigungen und können beispielsweise aus Papier, Pergament oder anderen dem Fachmann geläufigen Materialien gebildet sein. Stabile Metallfolien, welche üblicherweise zur Abdichtung von Vorratsbehältern mit darin gelagerten diagnostischen Testelementen verwendet werden, können auf diese Weise effizient und kostensparend vermieden werden.

Durch den Verzicht auf Dichtungselemente, welche das Eindringen von Feuchtigkeit aus der Umgebung in den Vorratsbehälter im wesentlichen verhindern, können Herstellung und Lagerung der hierin beschriebenen Testelemente erheblich vereinfacht und wirtschaftlicher gestaltet werden. So führt der Verzicht auf mechanische Bauteile zum einen zu einer erheblichen Verringerung des Bauvolumens gegenüber herkömmlichen Vorratsbehältern. Andererseits werden durch den Verzicht auf Dichtungselemente auch jene Zugkräfte, welche beispielsweise im Falle von Bandmagazinen mit Dichtungselement für den Transport von unverbrauchten Testbereichen eines in Form eines Testbandes vorliegenden diagnostischen Testelements aus einer Lagerungsposition innerhalb des Bandmagazins zu einer Probenaufgabeposition bzw. Messposition außerhalb des Bandmagazins infolge eines durch das Dichtungselement hervorgerufenen mechanischen Widerstandes benötigt werden, deutlich erniedrigt.

Insofern sieht eine bevorzugte Ausführungsform des erfindungsgemäßen Vorratsbehälters vor, dass die zum Transport von unverbrauchten Testbereichen eines diagnostischen Testelements aus einer Lagerungsposition innerhalb des Vorratsbehälters zu einer Probenaufgabeposition oder/und Messposition außerhalb des Vorratsbehälters oder/und die zum Transport von verbrauchten Testbereichen des diagnostischen Testelements von einer Probenaufgabeposition oder/und Messposition außerhalb des Vorratsbehälters zu einer Lagerungsposition innerhalb des Vorratsbehälters benötigte minimale oder/und maximale Zugkraft, bezogen auf einen entsprechenden Vorratsbehälter mit Dichtungselement, vermindert ist. Die Verringerung der minimalen oder/und maximalen Zugkraft beträgt hierbei üblicherweise mindestens 10%, bevorzugt mindestens 25%, und am stärksten bevorzugt mindestens 50%, bezogen auf die in einem entsprechenden Vorratsbehälter mit Dichtungselement benötigte minimale oder/und maximale Zugkraft.

Durch Verringerung der minimalen oder/und maximalen Zugkraft für den Weitertransport der Testbereiche eines diagnostischen Testelements in einem Vorratsbehälter der vorliegenden Erfindung kann beispielsweise im Falle von Bandmagazinen der Energiebedarf für den Weitertransport eines Testbandes erniedrigt werden, was zu einem insgesamt verringerten Energiebedarf eines den Vorratsbehälter umfassenden analytischen Messgeräts führt und damit beispielsweise den Einsatz kleinerer bzw. einer geringeren Anzahl an Batterien ermöglicht. Dies führt wiederum zu kleineren Bauräumen, so dass insgesamt eine signifikante Reduzierung der Größe der Vorratsbehälter, und damit eine Reduzierung der zur Herstellung der Vorratsbehälter benötigten Materialien, erzielt werden kann.

In einer zweiten Ausführungsform ermöglicht der erfindungsgemäße Vorratsbehälter einen Feuchtigkeitsaustausch zwischen unverbrauchten Testbereichen und verbrauchten Testbereichen eines einzelnen diagnostischen Testelements oder mehrerer diagnostischer Testelemente. Bevorzugt kommen in derartigen Vorratsbehältern diagnostische Testelemente zum Einsatz, die mehr als nur einen Testbereich zur Bestimmung eines Analyten umfassen, wie beispielsweise Testbänder.

Die Lagerung von unverbrauchten Testbereichen und verbrauchten Testbereichen eines in vorliegender Anmeldung beschriebenen diagnostischen Testelements kann gemäß der zweiten Ausführungsform in einer beliebigen Art und Weise erfolgen, sofern zwischen den unverbrauchten Testbereichen und den verbrauchten Testbereichen ein Feuchtigkeitsaustausch möglich ist. So können unverbrauchte Testbereiche und verbrauchte Testbereiche beispielsweise in einem Vorratsbehälter gelagert sein, welcher einen oder mehrere Lagerungsbereiche, insbesondere eine, zwei oder mehr als zwei Kammern, umfasst.

In dem Vorratsbehälter der zweiten Ausführungsform sind die unverbrauchten Testbereiche und die verbrauchten Testbereiche des diagnostischen Testelements in einer einzigen Kammer oder in zwei durch eine feuchtigkeitsdurchlässige Trennwand voneinander getrennten Kammern gelagert. Sofern unverbrauchte Testbereiche und verbrauchte Testbereiche in einer einzigen Kammer aufgenommen sind, so sind die unverbrauchten Testbereiche und die verbrauchten Testbereiche innerhalb der einen Kammer bevorzugt derart angeordnet, dass ein Feuchtigkeitsaustausch zwischen unverbrauchten Testbereichen und verbrauchten Testbereichen möglich ist, zwecks Vermeidung einer Kontamination unverbrauchter Testbereiche jedoch kein direkter Kontakt zwischen unverbrauchten Testbereichen und verbrauchten Testbereichen des diagnostischen Testelements stattfindet. Durch die vereinfachte Ausgestaltung des Vorratsbehälters kann dieser wiederum miniaturisiert, einfacher produziert und kostengünstiger bereitgestellt werden.

Erfindungsgemäß können die hierin beschriebenen Vorratsbehälter auch Kombinationen der oben beschriebenen ersten und zweiten Ausführungsform umfassen. Insofern ist eine weitere Variante der Erfindung auf einen Vorratsbehälter, enthaltend mindestens ein diagnostisches Testelement, welches ein Enzym und ein stabilisiertes Coenzym umfasst, gerichtet, wobei der Vorratsbehälter (a) im wesentlichen frei von Trocknungsmitteln ist, (b) keine Dichtungselemente umfasst, welche das Eindringen von Feuchtigkeit aus der Umgebung in den Vorratsbehälter im wesentlichen verhindern, oder/und (c) einen Feuchtigkeitsaustausch zwischen unverbrauchten Testbereichen und verbrauchtem Testbereichen des Testelements oder der Testelemente ermöglicht.

Ferner können die hierin beschriebenen Vorratsbehälter indessen auch physikalische Mittel umfassen, welche das Eindringen von partikulären Stoffen aus der Umgebung in den Vorratsbehälter verhindern, das Eindringen von Feuchtigkeit hingegen gestatten. Vorzugsweise ist hierbei im Falle von diagnostischen Testelementen mit mehr als einem Testbereich, wie beispielsweise eines Testbandes, der Weitertransport der Testbereiche in dem Vorratsbehälter im Vergleich zu einem Vorratsbehälter ohne die entsprechenden physikalischen Mittel nicht beeinträchtigt, beispielsweise durch Erhöhung der zum Weitertransport der Testbereiche in dem Vorratsbehälter benötigten minimalen oder/und maximalen Zugkraft, wie oben beschrieben.

Die hierin beschriebenen Vorratsbehälter enthalten obligatorisch mindestens ein diagnostisches Testelement. Diagnostische Testelemente, welche im Rahmen der vorliegenden Erfindung Anwendung finden können, umfassen grundsätzlich jedes dem Fachmann bekannte Testelement, welches zur Bestimmung eines Analyten geeignet ist und in einem erfindungsgemäßen Vorratsbehälter gelagert werden kann. Der Begriff "Lagerung" bedeutet in diesem Zusammenhang, dass das diagnostische Testelement für eine beliebige Zeitspanne, bevorzugt über einen Zeitraum von mindestens 2 Wochen, stärker bevorzugt über einen Zeitraum von mindestens 3 Monaten, noch stärker bevorzugt über einen Zeitraum von mindestens 6 Monaten, und am stärksten bevorzugt über einen Zeitraum von mindestens 12 Monaten in dem Vorratsbehälter aufgenommen ist, wobei die Lagerung vorzugsweise bei Atmosphärendruck, Raumtemperatur (25°C) und einer relativen Luftfeuchtigkeit von mindestens 50% erfolgt.

Vorzugsweise werden im Rahmen der vorliegenden Erfindung diagnostische Testelemente eingesetzt, auf die der Analyt in Form einer wässrigen oder nichtwässrigen Lösung aufgebracht werden kann. In einer bevorzugten Ausführungsform der Erfindung handelt es sich bei dem Testelement um ein Testband, eine Testdisc, ein Testpad, einen Teststreifen oder um die in WO 2005/084530 A2 genannten diagnostischen Elemente. Sofern die in vorliegender Anmeldung beschriebenen Vorratsbehälter als Teststreifenmagazin ausgebildet sind, so umfassen sie in einer besonders bevorzugten Variante eine Vielzahl miteinander verbundener, einzeln abtrennbarer Teststreifen in riegelförmiger oder/und rollenförmiger Anordnung. Falls gewünscht, können die diagnostischen Testelemente zusätzlich eine Markierung wie beispielsweise einen Barcode umfassen, welche bei Bedarf eine nachträgliche Identifizierung des jeweils verwendeten Testelements ermöglicht.

Der Begriff "Testband", wie er hierin verwendet wird, bezeichnet ein bandförmiges Testelement, welches üblicherweise mehr als einen einzelnen Testbereich, bevorzugt mindestens 10 einzelne Testbereiche, stärker bevorzugt mindestens 25 einzelne Testbereiche, und am stärksten bevorzugt mindestens 50 einzelne Testbereiche umfasst. In einer Ausführungsform sind die einzelnen Testbereiche jeweils in einem Abstand von wenigen Millimetern bis zu wenigen Zentimetern, beispielsweise in einem Abstand von < 2.5 cm, voneinander angeordnet, wobei das Testband zwischen aufeinander folgenden Testbereichen gegebenenfalls Markierungsbereiche zur Wegerfassung beim Bandtransport oder/und zur Kalibrierung umfassen kann und üblicherweise nicht mit Testchemie beschichtet ist. Testbänder mit Markierungsbereichen zur Wegerfassung beim Bandtransport zwischen aufeinander folgenden Testbereichen sind beispielsweise in EP 1 739 432 A1 beschrieben.

Vorzugsweise handelt es sich bei dem oben beschriebenen Testband um ein durchgängiges Testband, bei welchem mindestens zwei Testbereiche direkt nebeneinander, d. h. ohne Zwischenraum zwischen den zwei Testbereichen, angeordnet sind. Eine derartige Anordnung von Testbereichen hat den Vorteil, dass eine größere Anzahl von Testbereichen auf dem Testband positioniert werden kann und darüber hinaus ein Feuchtigkeitsaustausch zwischen den einzelnen Testbereichen ermöglicht wird. Auf diese Weise kann die Herstellung von Testbändern sowohl vereinfacht als auch kostengünstiger gestaltet werden.

Bei einem durchgängigen Testband im Sinne der vorliegenden Erfindung können beispielsweise alle Testbereiche mit Ausnahme des ersten und letzten Testbereiches derart angeordnet sein, dass ein einzelner Testbereich auf beiden Seiten direkt an einen weiteren Testbereich angrenzt. Alternativ können bei einem durchgängigen Testband auch jeweils mehrere Testbereiche, beispielsweise 5 Testbereiche oder mehr, direkt nebeneinander angeordnet sein, wobei aufeinander folgende Gruppen von Testbereichen ihrerseits in einem Abstand von wenigen Millimetern bis zu wenigen Zentimetern, beispielsweise in einem Abstand von < 2.5 cm, voneinander angeordnet sind. In diesem Fall kann das Testband zwischen aufeinander folgenden Gruppen von Testbereichen gegebenenfalls ebenfalls Markierungsbereiche zur Wegerfassung beim Bandtransport oder/und zur Kalibrierung umfassen.

Der Begriff "Testdisc", wie er hierin verwendet wird, bezeichnet ein scheibenförmiges Testelement, welches einen oder mehrere einzelne Testbereiche, beispielsweise mindestens 10 einzelne Testbereiche, umfassen kann. In einer Ausführungsform ist die Testdisc mit einer dünnen Schicht der Testchemie, beispielsweise mit einer Schicht in einer Dicke von etwa 20 µm, überzogen, auf welche eine Probe des Analyten aufgebracht werden kann, wobei in Abhängigkeit vom Volumen der Probe ein mehr oder weniger großer Bereich der Testdisc von der Probe benetzt wird und zur Bestimmung des Analyten verwendet werden kann. Der nicht-benetzte Bereich der Testdisc, welcher infolge des Durchtritts von Feuchtigkeit durch die Testchemieschicht teilweise oder vollständig befeuchtet sein kann, steht in der Folge für weitere Bestimmungen des Analyten zur Verfügung.

Das in dem diagnostischen Testelement eingesetzte Enzym kann ein beliebiges Enzym sein, welches dem Fachmann für die Zwecke der jeweiligen Anwendung geeignet erscheint, z. B. eine Dehydrogenase. Vorzugsweise handelt es sich bei dem Enzym um eine Nikotinamid-Adenin-Dinukleotid (NAD/NADH)- oder Nikotinamid-Adenin-Dinukleotidphosphat (NADP/NADPH)-abhängige Dehydrogenase, welche insbesondere ausgewählt ist aus einer Alkohol-Dehydrogenase (EC 1.1.1.1; EC 1.1.1.2), einer L-Aminosäure-Dehydrogenase (EC 1.4.1.5), einer Glucose-Dehydrogenase (EC 1.1.1.47), einer Glucose-6-phosphat-Dehydrogenase (EC 1.1.1.49), einer Glycerin-Dehydrogenase (E.C.1.1.1.6), einer 3-Hydroxybutyrat-Dehydrogenase (EC 1.1.1.30), einer Lactat-Dehydrogenase (EC 1.1.1.27; 1.1.1.28), einer Malat-Dehydrogenase (EC 1.1.1.37) und einer Sorbitol-Dehydrogenase. In der am stärksten bevorzugten Ausführungsform handelt es sich bei dem Enzym um eine Glucose-Dehydrogenase (EC 1.1.1.47) oder eine Glucose-6-phosphat-Dehydrogenase (EC 1.1.1.49).

Ein stabilisiertes Coenzym im Sinne der vorliegenden Erfindung ist ein gegenüber dem nativen Coenzym chemisch verändertes Coenzym, welches bei Atmosphärendruck eine im Vergleich zum nativen Coenzym höhere Stabilität gegenüber Feuchtigkeit, Temperaturen insbesondere im Bereich von 0°C bis 50°C, Säuren und Basen insbesondere im Bereich von pH 4 bis pH 10, oder/und Nukleophilen wie beispielsweise Alkoholen oder Aminen, aufweist und insofern unter identischen Umgebungsbedingungen über einen längeren Zeitraum als das native Coenzym seine Wirkung entfalten kann. Vorzugsweise weist das stabilisierte Coenzym eine im Vergleich zum nativen Coenzym höhere hydrolytische Stabilität auf, wobei eine vollständige Hydrolysestabilität unter Testbedingungen besonders bevorzugt ist. Im Vergleich zum nativen Coenzym kann das stabilisierte Coenzym eine verringerte Bindungskonstante für das Enzym aufweisen, beispielsweise eine um den Faktor von 2 oder mehr verringerte Bindungskonstante.

Ein bevorzugtes Beispiel für stabilisierte Coenzyme im Sinne der vorliegenden Erfindung sind stabilisierte NAD(P)/NAD(P)H-Verbindungen, d. h. chemische Derivate von nativem Nikotinamid-Adenin-Dinukleotid (NAD/NADH) bzw. Nikotinamid-Adenin-Dinukleotidphosphat (NADP/NADPH). Bevorzugt umfasst die stabilisierte NAD(P)/NAD(P)H-Verbindungen einen 3-Pyridincarbonyl- oder einen 3-Pyridinthiocarbonyl-Rest, welcher ohne glykosidische Bindung über einen linearen oder cyclischen organischen Rest, insbesondere über einen cyclischen organischen Rest, mit einem phosphorhaltigen Rest, wie beispielsweise einem Phosphatrest, verknüpft ist.

Besonders bevorzugt wird die stabilisierte NAD(P)/NAD(P)H-Verbindung aus Verbindungen der allgemeinen Formel (I) ausgewählt: 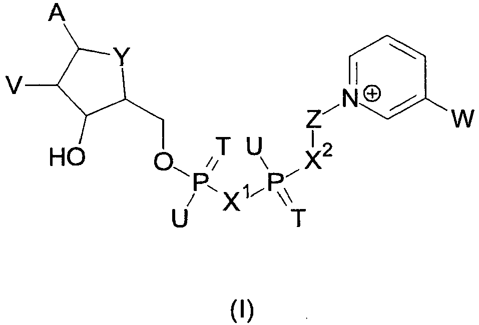 worin
- A =: Adenin oder ein Analog davon,
- T =: jeweils unabhängig O, S,
- U =: jeweils unabhängig OH, SH, BH₃⁻, BCNH₂⁻,
- V =: jeweils unabhängig OH oder eine Phosphatgruppe, oder zwei Gruppen, die eine cyclische Phosphatgruppe bilden;
- W =: COOR, CON(R)₂, COR, CSN(R)₂ mit R = jeweils unabhängig H oder C₁-C₂-Alkyl,
- X¹, X² =: jeweils unabhängig O, CH₂, CHCH₃, C(CH₃)₂, NH, NCH₃,
- Y =: NH, S, O, CH₂,
- Z =: ein linearer oder cyclischer organischer Rest ist,
mit der Maßgabe, dass Z und der Pyridin-Rest nicht durch eine glycosidische Verbindung verknüpft sind, oder ein Salz oder gegebenenfalls eine reduzierte Form davon.

In den Verbindungen der Formel (I) ist Z vorzugsweise ein linearer Rest mit 4-6 C-Atomen, vorzugsweise mit 4 C-Atomen, worin 1 oder 2 C-Atome gegebenenfalls durch ein oder mehrere Heteroatome ausgewählt aus O, S und N ersetzt sind, oder ein Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest CR⁴₂, wobei CR⁴₂ an die cyclische Gruppe und an X² gebunden ist, mit R⁴ = jeweils unabhängig H, F, Cl, CH₃.

Besonders bevorzugt ist Z ein gesättiger oder ungesättigter carbocyclischer oder heterocyclischer Fünfring, insbesondere eine Verbindung der allgemeinen Formel (II), 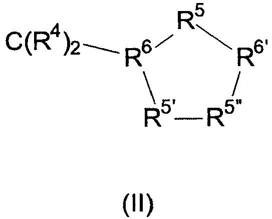 wobei zwischen R⁵' und R⁵" eine Einfach- oder Doppelbindung vorliegen kann, mit
- R⁴ =: jeweils unabhängig H, F, Cl, CH₃,
- R⁵ =: CR⁴₂,
- R⁵' =: O, S, NH, NC₁-C₂-Alkyl, CR⁴₂, CHOH, CHOCH₃, und R⁵" = CR⁴₂, CHOH, CHOCH₃, wenn zwischen R⁵' und R⁵" eine Einfachbindung vorliegt,
- R⁵' = R⁵" = CR⁴,: wenn zwischen R⁵' und R⁵" eine Doppelbindung vorliegt, und
- R⁶, R⁶' =: jeweils unabhängig CH oder CCH₃.

In einer bevorzugten Ausführungsform enthalten die erfindungsgemäßen Verbindungen Adenin oder Adenin-Analoga, wie z.B. C₈- und N₆-substituiertes Adenin, Deaza-Varianten wie 7-Deaza, Azavarianten wie 8-Aza oder Kombinationen wie 7-Deaza oder 8-Aza oder carbocyclische Analoga, wie Formycin, wobei die 7-Deazavarianten in der 7-Position mit Halogen, C₁₋₆-Alkinyl, -Alkenyl oder -Alkyl substituiert sein können.

In einer weiteren bevorzugten Ausführungsform enthalten die Verbindungen Adenosin-Analoga, welche statt Ribose z.B. 2-Methoxydesoxyribose, 2'-Fluorodesoxyribose, Hexitol, Altritol bzw. polycyclische Analoga, wie Bicyclo-, LNA- und Tricyclo-Zucker enthalten.

Insbesondere können in den Verbindungen der Formel (I) auch (Di-)-Phosphatsauerstoffe isotronisch ersetzt sein, wie z.B. O⁻ durch S⁻ bzw. BH₃⁻, O durch NH, NCH₃ bzw. CH₂ und =O durch =S. In den erfindungsgemäßen Verbindungen der Formel (I) ist W vorzugsweise CONH₂ oder COCH₃.

In den Gruppen der Formel (II) ist R⁵ vorzugsweise CH₂. Weiterhin ist bevorzugt, dass R⁵' ausgewählt ist aus CH₂, CHOH und NH. In einer besonders bevorzugten Ausführungsform sind R⁵' und R⁵" jeweils CHOH. In noch einer weiteren bevorzugten Ausführungsform ist R⁵' NH und R⁵" CH₂. Am stärksten bevorzugt ist eine Verbindung der Formel (II), in welcher R⁴ = H ist, R⁵ = CH₂ ist, R⁵' = R⁵" = CHOH ist, und R⁶ = R⁶' = CH ist.

In der am stärksten bevorzugten Ausführungsform handelt es sich bei dem stabilisierten Coenzym um die literaturbekannte Verbindung carbaNAD (J.T. Slama, Biochemistry (1988), 27, 183 und Biochemistry (1989), 28, 7688). Andere stabile Coenzyme, welche erfindungsgemäß Anwendung finden können, sind in WO 98/33936, WO 01/49247, WO 2007/012494, US 5,801,006, US 11/460,366 und der Publikation Blackburn et al. (Chem. Comm. (1996), 2765), beschrieben.

Das im Rahmen der vorliegenden Anmeldung beschriebene diagnostische Testelement kann ein beliebiges Testelement sein, welches eine das Enzym und das stabilisierte Coenzym enthaltende trockene Reagenzienschicht umfasst und von der den Analyten enthaltenden Probe benetzt werden kann. Die Reagenzienschicht kann neben dem Enzym und dem stabilisierten Coenzym ggf. weitere Reagenzien, welche dem qualtitativen Nachweis oder der quantitativen Bestimmung des Analyten dienen, wie beispielsweise Mediatoren, optische Indikatoren, sowie geeignete Hilfs- oder/und Zusatzstoffe umfassen.

Der Begriff "Mediator", wie er im Rahmen dieser Anmeldung verwendet wird, bedeutet eine chemische Verbindung, welche das durch Umsetzung mit dem Analyten erhaltene, reduzierte Coenzym oxidiert und hierbei die Reaktivität des Coenzyms erhöht. Als Mediatoren, welche erfindunsgemäß eingesetzt werden können, kommen u.a. Nitrosoaniline, wie beispielsweise [(4-Nitrosophenyl)imino]dimethanol-Hydrochlorid, Chinone, wie beispielsweise ein Phenanthrenchinon, ein Phenanthrolinchinon oder ein Benzo[h]-chinolinchinon, Phenazine, wie beispielsweise 1-(3-Carboxypropoxy)-5-ethylphenazinium-trifluormethansulfonat, oder/und Diaphorase (EC 1.6.99.2) in Frage.

Bevorzugt wird als Mediator ein Chinon, insbesondere ein 1,10-Phenanthrolinchinon, ein 1,7-Phenanthrolinchinon, ein 4,7-Phenanthrolinchinon, oder N-alkylierte oder N,N'-dialkylierte Salze hiervon, eingesetzt. Als besonders vorteilhaft haben sich in diesem Zusammenhang N-Methyl-1,10-Phenanthrolinium-5,6-chinon, N,N'-Dimethyl-1,10-Phenanthrolinium-5,6-chinon, N-Methyl-1,7-Phenanthrolinium-5,6-chinon, N,N'-Dimethyl-1,7-Phenanthrolinium-5,6-chinon, N-Methyl-4,7-Phenanthrolinium-5,6-chinon und N,N'-Dimethyl-4,7-Phenanthrolinium-5,6-chinon erwiesen, wobei im Falle N-alkylierter bzw. N,N'-dialkylierter Salze ein beliebiges Anion als Gegenion des Mediators fungieren kann. In einer bevorzugten Ausführungsform wird als Gegenion ein Halogenid oder Trifluormethansulfonat eingesetzt.

Als optischer Indikator kann eine beliebige Substanz verwendet werden, welche reduzierbar ist und bei Reduktion eine detektierbare Änderung ihrer optischen Eigenschaften, wie beispielsweise Farbe, Fluoreszenz, Remission, Transmission, Polarisation oder/und Brechungsindex, erfährt. Die Bestimmung des Vorhandenseins oder/und der Menge des Analyten in der Probe kann mit dem bloßen Auge oder/und mittels einer Detektionsvorrichtung unter Verwendung eines dem Fachmann geeignet erscheinenden optischen, insbesondere photometrischen oder fluorimetrischen, oder elektrochemischen Verfahrens erfolgen. Vorzugsweise werden Heteropolysäuren, und insbesondere 2,18-Phosphormolybdänsäure, als optische Indikatoren verwendet, die zum entsprechenden Heteropolyblau reduziert werden.

In einer bevorzugten Ausführungsform der Erfindung ist neben dem Vorratsbehälter auch das diagnostische Testelement im wesentlichen frei von Trocknungsmitteln. In Bezug auf diagnostische Testelemente bedeutet der Ausdruck "im wesentlichen frei von Trocknungsmitteln", dass das diagnostische Testelement eine Menge von < 5 Gew.%, bevorzugt eine Menge von < 1 Gew.%, stärker bevorzugt eine Menge von < 0.1 Gew.%, noch stärker bevorzugt eine Menge von < 0.01 Gew.%, und am stärksten bevorzugt eine Menge von < 0.001 Gew.% an Trocknungsmittel, bezogen auf das Gesamtgewicht der Testchemie des diagnostischen Testelements, enthält. Die Möglichkeit einer Verringerung der Menge an Trocknungsmittel bzw. eines vollständigen Verzichts auf Trocknungsmittel im Rahmen der Herstellung und Lagerung der hierin beschriebenen Testelemente hat den Vorteil, dass sowohl Herstellung als auch Lagerung der Testelemente vereinfacht und damit kostengünstiger bewerkstelligt werden kann.

In einer weiteren bevorzugten Ausführungsform umfassen die erfindungsgemäß eingesetzten diagnostischen Testelemente neben dem Enzym und dem stabilisierten Coenzym weiterhin ein Nadelelement zum Anritzen von Haut, welches aus einem beliebigen Material, insbesondere jedoch aus Metall oder Kunststoff, bestehen kann und vorzugsweise in das diagnostische Testelement integriert ist. Unter einem integrierten Nadelelement im Sinne der vorliegenden Erfindung ist ein Nadelelement zu verstehen, welches physikalisch mit dem diagnostischen Testelement verbunden ist. Im Gegensatz hierzu ist ein separates Nadelelement als ein vom diagnostischen Testelement getrennt vorliegendes Nadelelement definiert, welches keine physikalische Verbindung mit dem diagnostischen Testelement aufweist.

Erfindungsgemäß dient das Nadelelement zum Anritzen von Haut, wobei Körperfluide wie beispielsweise Blut freigesetzt werden, welche mittels der der hierin beschriebenen diagnostischen Testelemente auf einen Analyten hin untersucht werden können. Das freigesetzte Körperfluid kann hierbei direkt oder indirekt, beispielsweise unter Verwendung eines Probennahmeelements, auf das diagnostische Testelement transferiert werden. Als Probennahmeelement kann dabei ein beliebiges Element verwendet werden, welches eine zur Bestimmung des Analyten ausreichende Menge des Körperfluids aufzunehmen vermag und einen nachfolgenden Transfer zumindest eines Teils des aufgenommenen Körperfluids auf das diagnostische Testelement ermöglicht, wobei sich Kapillaren als besonders vorteilhaft erwiesen haben. Insofern umfasst das zum Anritzen von Haut verwendete Nadelelement in einer Variante der Erfindung zusätzlich einen Kapillarkanal, mittels welchem freigesetztes Körperfluid aufgenommen und unter Ausnutzung von Kapillarkräften auf das diagnostische Testelement transferiert werden kann.

Mittels des erfindungsgemäßen Vorratsbehälters besteht die Möglichkeit, die oben beschriebenen diagnostischen Testelemente für längere Zeit, gegebenenfalls bei einer relativen Luftfeuchtigkeit von mindestens 50% oder/und bei einer Temperatur von mindestens 20°C, ohne merklichen Verlust der Enzymaktivität zu lagern. Dies bedeutet, dass das diagnostische Testelement beispielsweise nach Lagerung über eine Dauer von mindestens 4 Wochen, vorzugsweise von mindestens 8 Wochen, und besonders bevorzugt von mindestens 12 Wochen bei einer Temperatur von mindestens 20°C, bevorzugt von mindestens 25°C, und besonders bevorzugt von mindestens 30°C eine Abnahme der Enzymaktivität von weniger als 50%, bevorzugt von weniger als 30%, und besonders bevorzugt von weniger als 20%, bezogen auf den Ausgangswert der Enzymaktivität, aufweist. Verfahren bzw. Tests zur Bestimmung der Aktivität von Enzymen sind im Stand der Technik weithin bekannt und können vom Fachmann, sofern erforderlich, den jeweiligen Bedürfnissen angepasst werden, wobei zum Vergleich der Enzymaktivität vor und nach der Lagerung jeweils die gleichen Testbedingungen verwendet werden.

Die mittels des erfindungsgemäßen Vorratsbehälters gelagerten diagnostischen Testelemente können zur Bestimmung einer beliebigen biologischen oder chemischen Substanz verwendet werden, welche photochemisch oder elektrochemisch nachweisbar ist. Vorzugsweise wird der Analyt aus der Gruppe bestehend aus Äpfelsäure, Alkohol, Ammonium, Ascorbinsäure, Cholesterin, Cystein, Glucose, Glutathion, Glycerin, Harnstoff, 3-Hydroxybutyrat, Milchsäure, 5'-Nukleotidase, Peptiden, Pyruvat, Salicylat und Triglyceriden ausgewählt, wobei Glucose besonders bevorzugt ist. Der Analyt kann hierbei aus einer beliebigen Quelle stammen, ist jedoch bevorzugt in einem Körperfluid, umfassend, jedoch nicht beschränkt auf, Vollblut, Plasma, Serum, Lymphflüssigkeit, Gallenflüssigkeit, Cerebrospinalflüssigkeit, extrazelluläre Gewebeflüssigkeit, Harn, sowie Drüsensekrete wie beispielsweise Speichel oder Schweiß, enthalten. Bevorzugt wird mittels der hierin beschriebenen diagnostischen Testelemente das Vorhandensein und/oder die Menge eines Analyten in einer Probe aus Vollblut, Plasma, Serum oder extrazellulärer Gewebeflüssigkeit bestimmt.

Die qualitative oder/und quantitative Bestimmung des Analyten kann auf beliebige Art und Weise erfolgen. Hierzu können grundsätzlich alle aus dem Stand der Technik bekannten Methoden zum Nachweis enzymatischer Reaktionen eingesetzt werden, welche ein messbares Signal erzeugen, das manuell oder unter Verwendung geeigneter Mittel ausgewertet bzw. ausgelesen werden kann. Im Rahmen der vorliegenden Erfindung gelangen bevorzugt optische Nachweismethoden, welche beispielsweise die Messung von Absorption, Fluoreszenz, Circulardichroismus (CD), optische Rotationsdispersion (ORD), Refraktometrie, etc. umfassen, sowie elektrochemische Techniken zur Anwendung. Besonders bevorzugt erfolgt der Nachweis des Analyten photometrisch oder fluorometrisch, beispielsweise indirekt über eine fluorometrisch detektierbare Veränderung des Coenzyms.

In einem weiteren Aspekt betrifft die Erfindung ein analytisches Messgerät, welches einen erfindungsgemäßen Vorratsbehälter oder ein erfindungsgemäßes diagnostisches Produkt umfasst und zur qualitativen oder/und quantitativen Bestimmung eines Analyten dient. Beispiele für derartige analytische Messgeräte umfassen u.a. die kommerziell erhältlichen Produkte Accu-Chek^{®} Active, Accu-Chek^{®} Compact und Accu-Chek^{®} Mobile (alle Fa. Roche), sind jedoch nicht auf diese beschränkt.

Die Erfindung soll durch die nachfolgenden Figuren und Beispiele näher erläutert werden:

### Beschreibung der Figuren

**Figur 1****:** Darstellung des stabilen Coenzyms carbaNAD (cNAD).
**Figur 2****:** Darstellung der Ergebnisse der Enzymkinetik von Glucose-Dehydrogenase in Gegenwart von NAD bzw. von Glucose-Dehydrogenase in Gegenwart von cNAD vor und nach Lagerung.
**2A:** Kinetik von GlucDH in Gegenwart von NAD nach 1 Tag.
**2B:** Kinetik von GlucDH in Gegenwart von cNAD nach 1 Tag.
**2C:** Kinetik von GlucDH in Gegenwart von NAD nach 5 Wochen Lagerung bei 32°C und 85% relativer Luftfeuchtigkeit.
**2D:** Kinetik von GlucDH in Gegenwart von cNAD nach 5 Wochen Lagerung bei 32°C und 85% relativer Luftfeuchtigkeit.

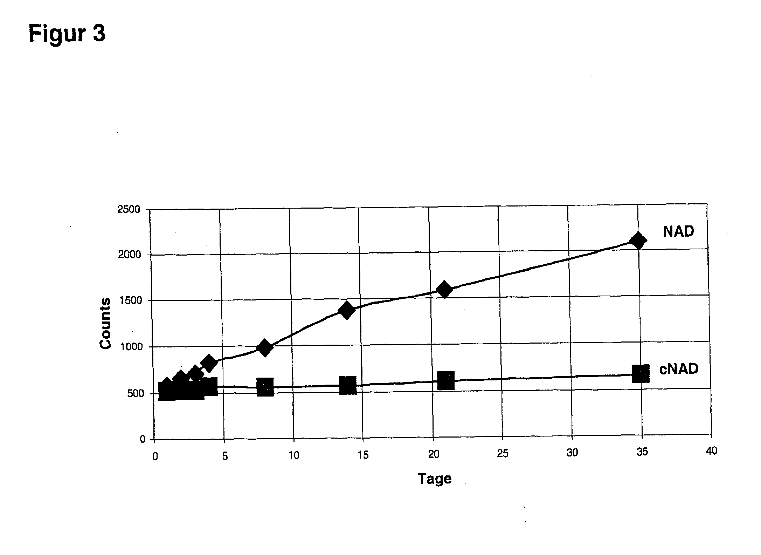

**Figur 3****:** Vergleich der Blankwerte von Glucose-Dehydrogenase in Gegenwart von NAD bzw. von GlucDH in Gegenwart von cNAD über einen Zeitraum von bis zu 5 Wochen bei 32°C und 85% Luftfeuchtigkeit.

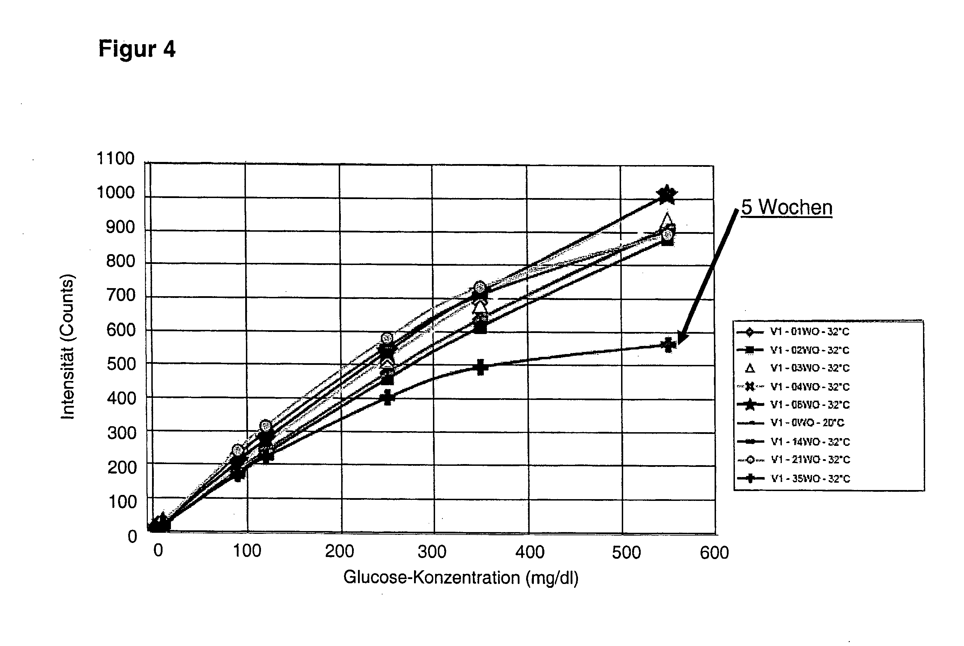

**Figur 4****:** Darstellung verschiedener Funktionskurven von Glucose-Dehydrogenase nach Lagerung von Glucose-Dehydrogenase in Gegenwart von NAD bei 32°C und 85% Luftfeuchtigkeit. Die Lagerungsdauer variierte zwischen 1 Tag und 5 Wochen.

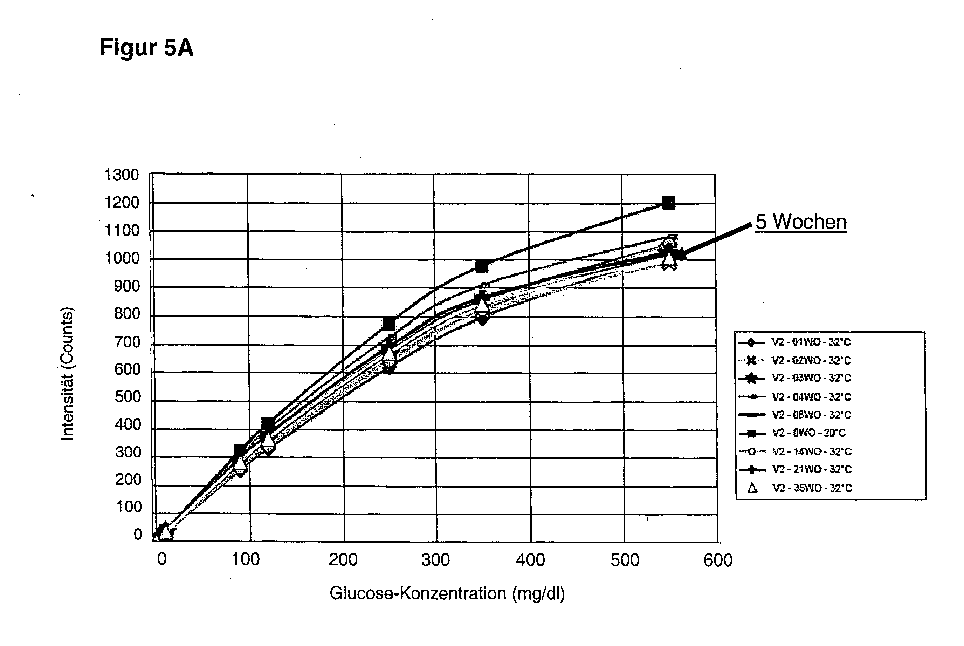

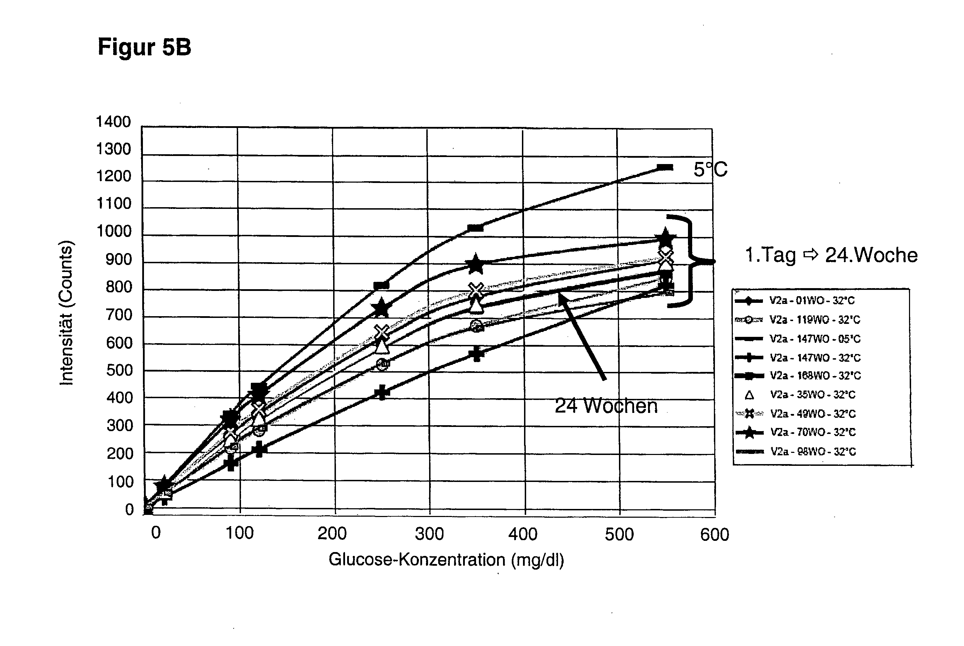

**Figur 5****:** Darstellung verschiedener Funktionskurven von Glucose-Dehydrogenase nach Lagerung von Glucose-Dehydrogenase in Gegenwart von cNAD bei 32°C und 85% Luftfeuchtigkeit. Die Lagerungsdauer variierte zwischen 1 Tag und 5 Wochen (Figur 5A) bzw. zwischen 1 Tag und 24 Wochen (Figur 5B).

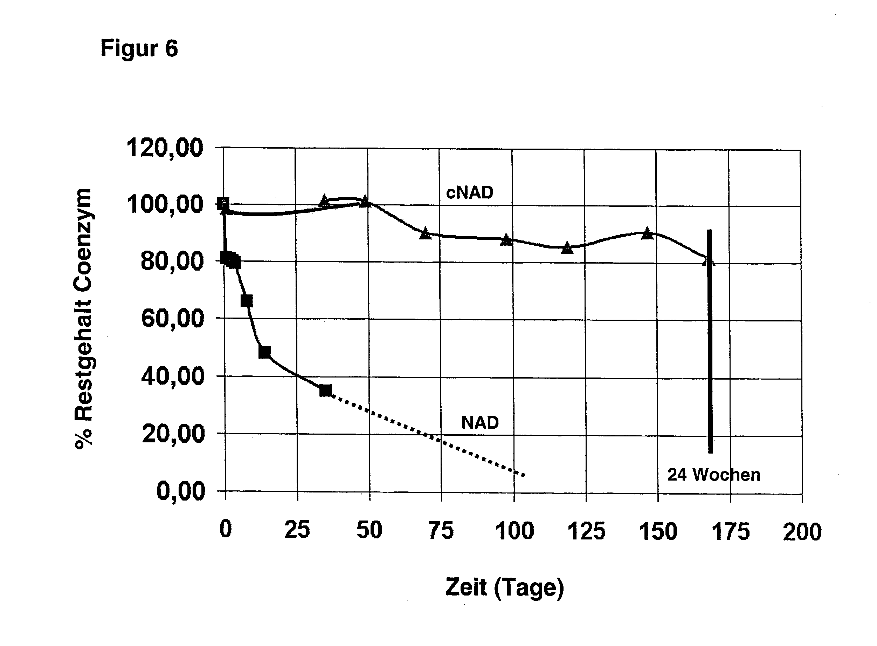

**Figur 6****:** Darstellung des Restgehaltes an NAD bzw. cNAD nach Lagerung von Glucose-Dehydrogenase in Gegenwart von NAD bzw. cNAD über 24 Wochen bei 32°C und 85% Luftfeuchtigkeit.

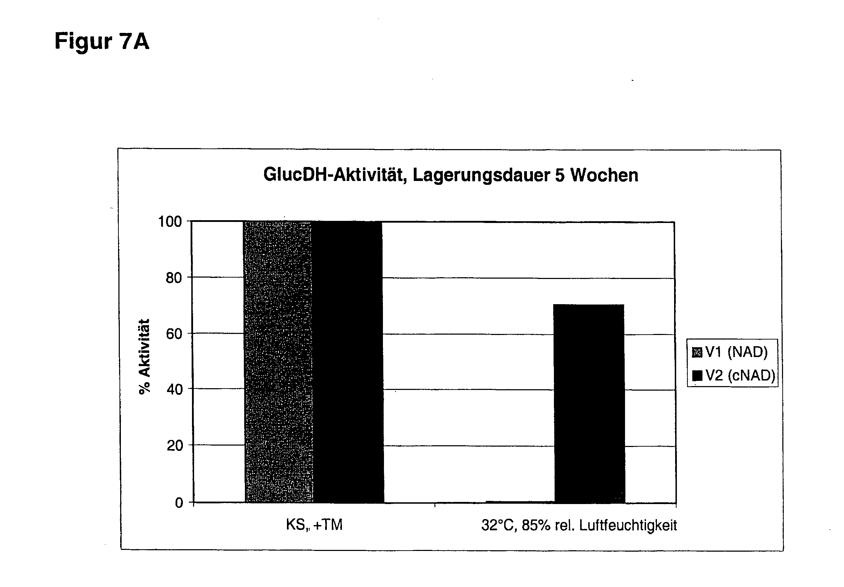

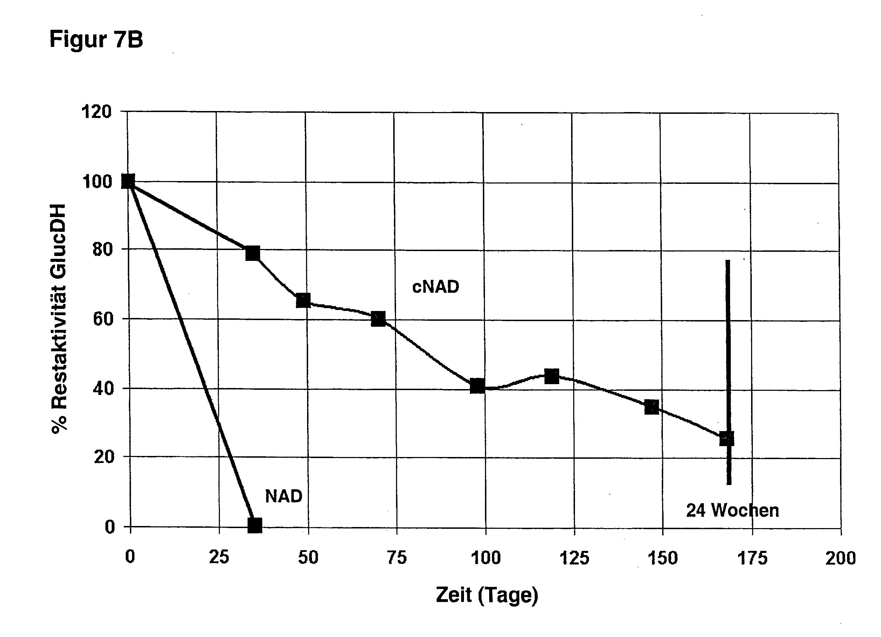

**Figur 7****:** Darstellung der GlucDH-Aktivität nach Lagerung von Glucose-Dehydrogenase in Gegenwart von NAD bzw. cNAD über 5 Wochen (Figur 7A) bzw. 24 Wochen (Figur 7B) bei 32°C und 85% Luftfeuchtigkeit.

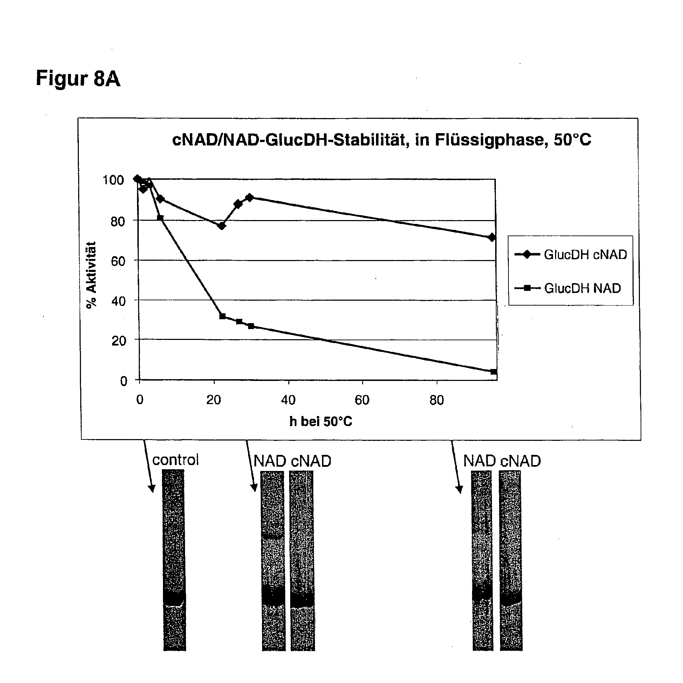

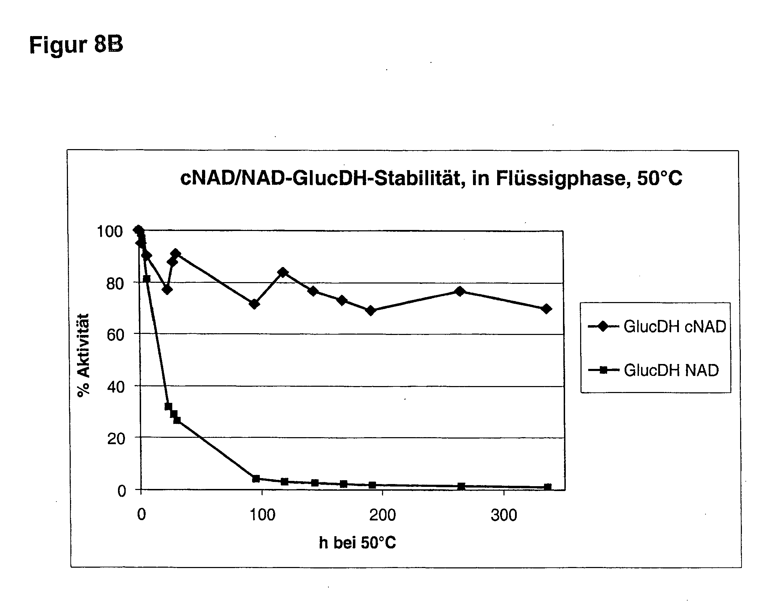

**Figur 8****:** Darstellung der Stabilität von Glucose-Dehydrogenase in Gegenwart von NAD bzw. cNAD in Flüssigphase bei 50°C über einen Zeitraum von 4 Tagen (Figur 8A) bzw. 14 Tagen (Figur 8B). Testbedingungen: GlucDH 10 mg/ml; NAD bzw. cNAD 12 mg/ml; Puffer: 0,1 M Tris, 1,2 M NaCl, pH 8,5; Temperatur 50°C.

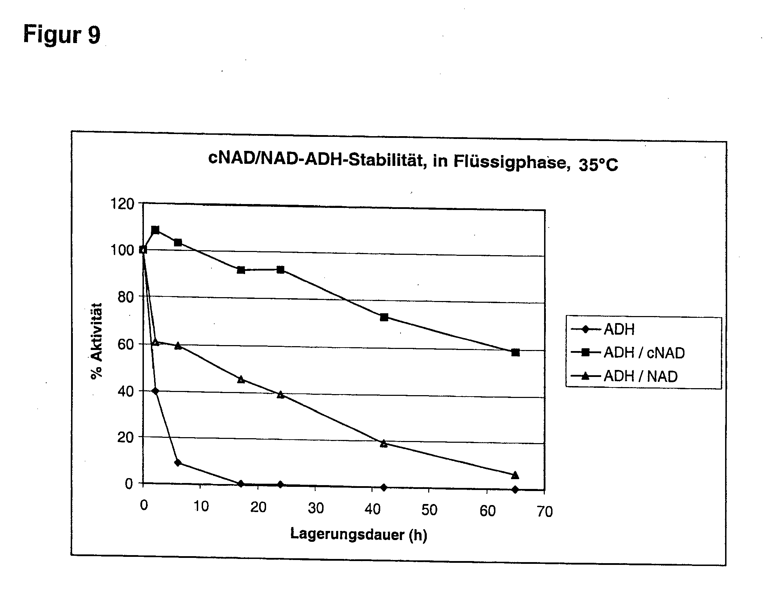

**Figur 9****:** Darstellung der Stabilität von Alkohol-Dehydrogenase aus Hefe in Gegenwart von NAD bzw. cNAD in Flüssigphase bei 35°C über einen Zeitraum von 65 Stunden. Testbedingungen: ADH 5 mg/ml; NAD bzw. cNAD 50 mg/ml; Puffer: 75 mM Na₄P₂O₇, Glycin, pH 9,0; Temperatur 35°C.

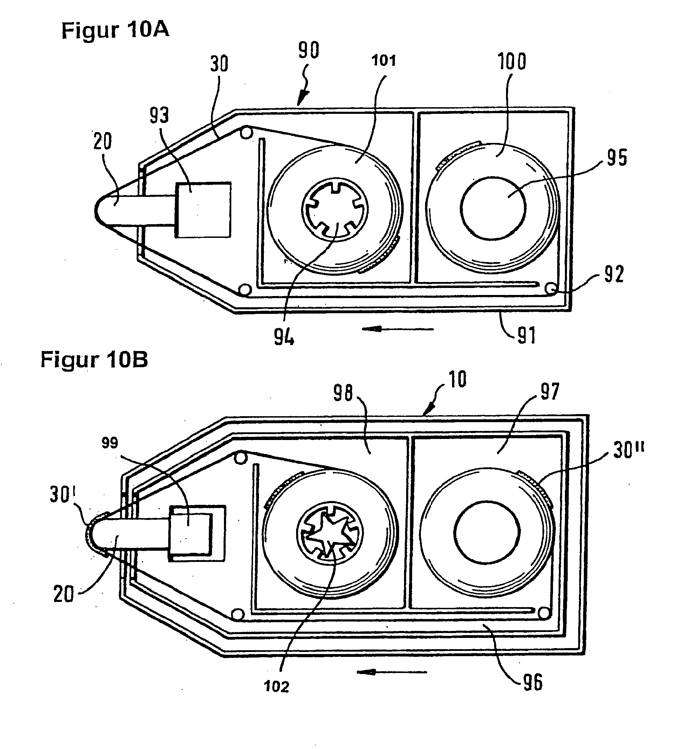

**Figur 10****:** Darstellung eines erfindungsgemäßen Vorratsbehälters in Form eines Bandmagazins mit zwei Kammern, in welchem ein Testband als diagnostisches Testelement verwendet wird (Figur 10A), bzw. eines den Vorratsbehälter enthaltenden analytischen Messgeräts (Figur 10B).

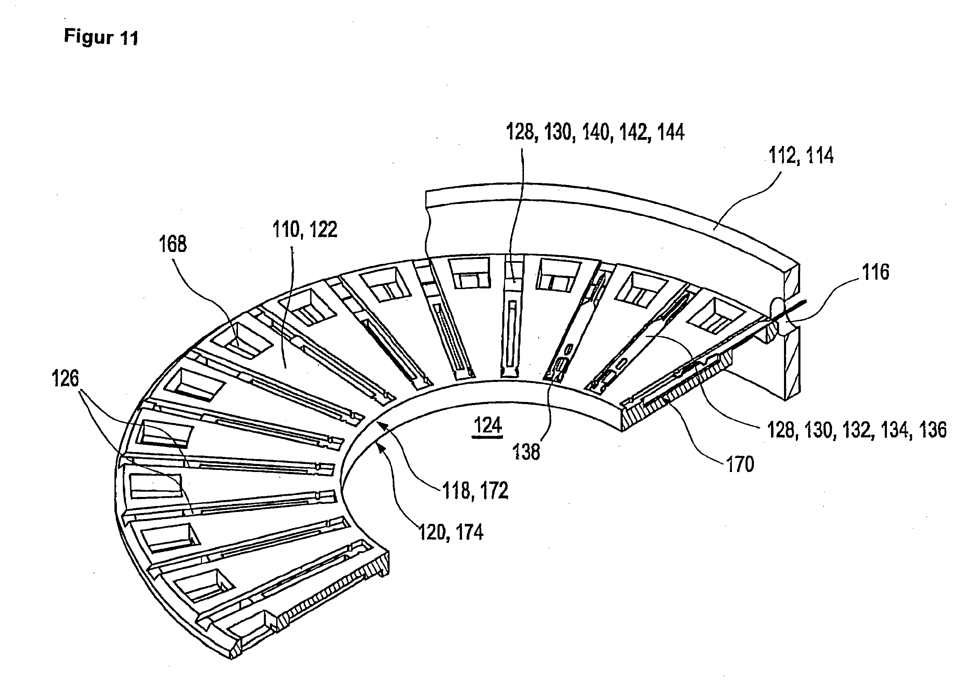

**Figur 11****:** Darstellung eines erfindungsgemäßen Vorratsbehälters in Form eines scheibenförmigen Wendemagazins, in welchem eine Vielzahl diagnostischer Testelemente aufgenommen ist.

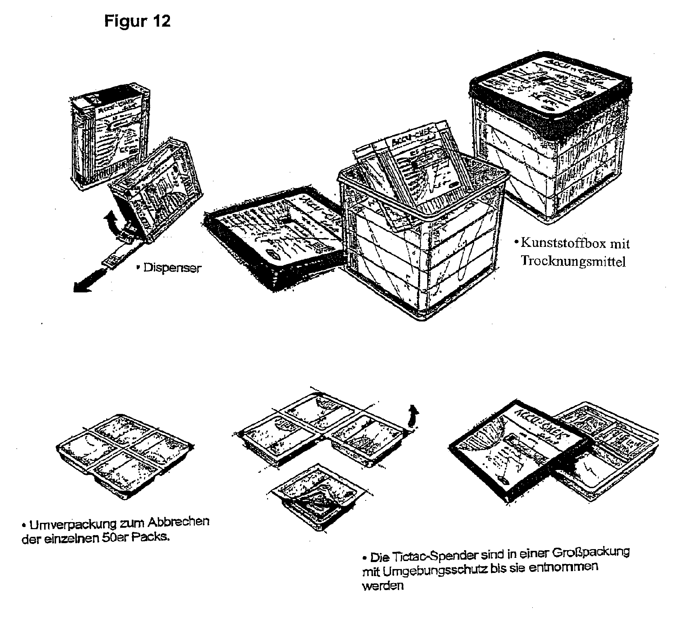

**Figur 12****:** Darstellung eines erfindungsgemäßen Vorratsbehälters in Form eines Tic Tac^{®}-Spenders, welcher eine Vielzahl einzelner Teststreifen als diagnostische Testelemente enthält und in einer mit Trockenmittel befüllten Umverpackung aufgenommen ist.

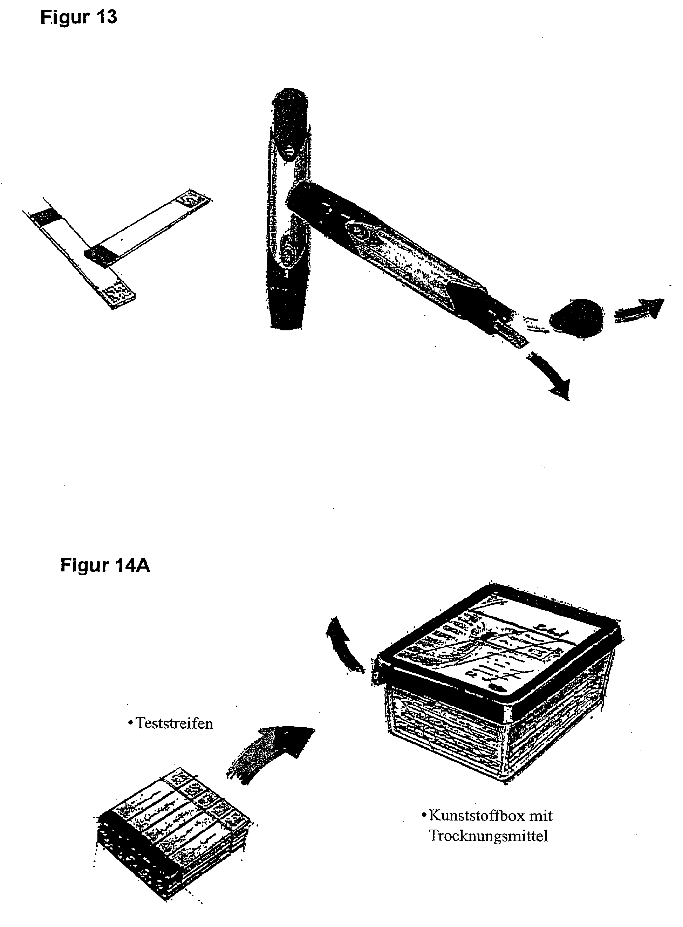

**Figur 13****:** Darstellung eines erfindungsgemäßen Vorratsbehälters in Form einer Stechhilfe, welche einen Einzelteststreifen als diagnostisches Testelement enthält.

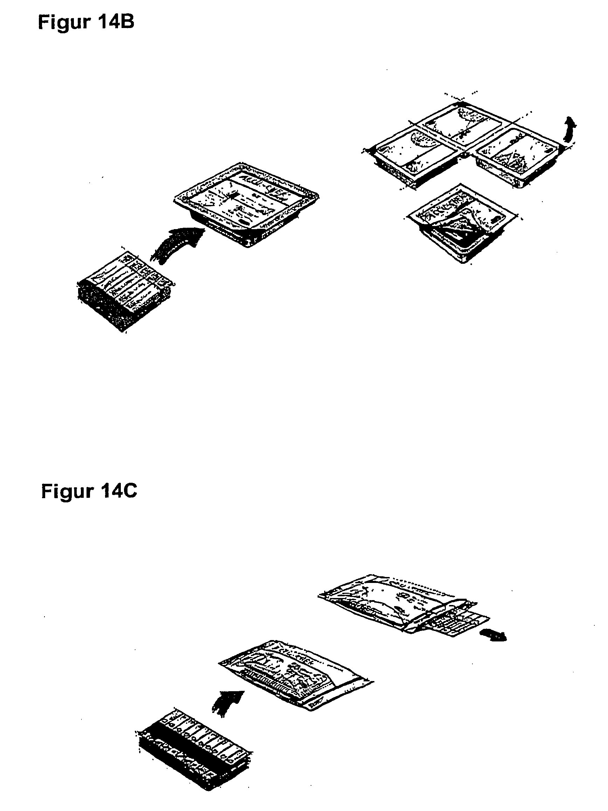

**Figur 14****:** Darstellung eines erfindungsgemäßen Vorratsbehälters in Form einer Kunststoffbox mit Trocknungsmittel (Figur 14A), einer Aluminiumdose (Figur 14B) bzw. eines Kunststoffbeutels (Figur 14C), welche jeweils eine Vielzahl miteinander verbundener, einzeln abtrennbarer Teststreifen in riegelförmiger Anordnung enthalten.

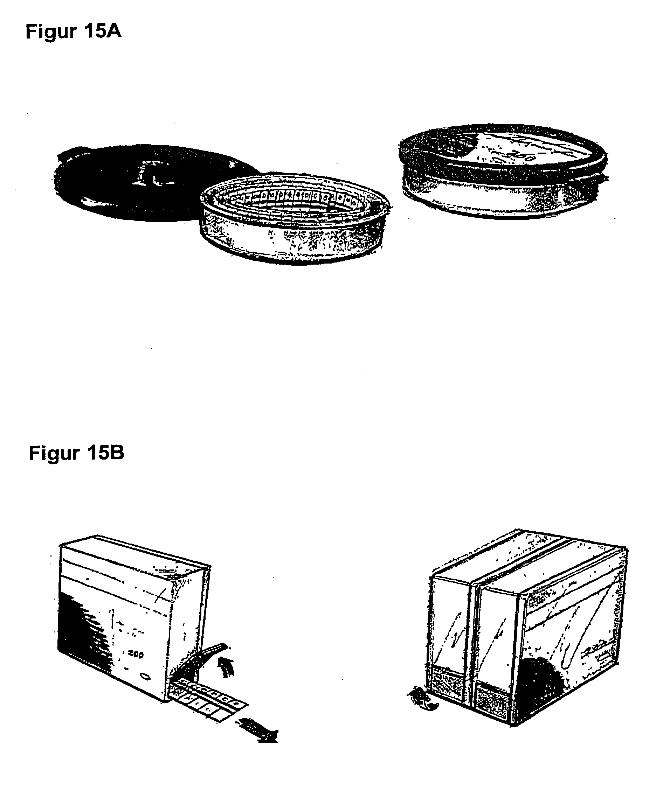

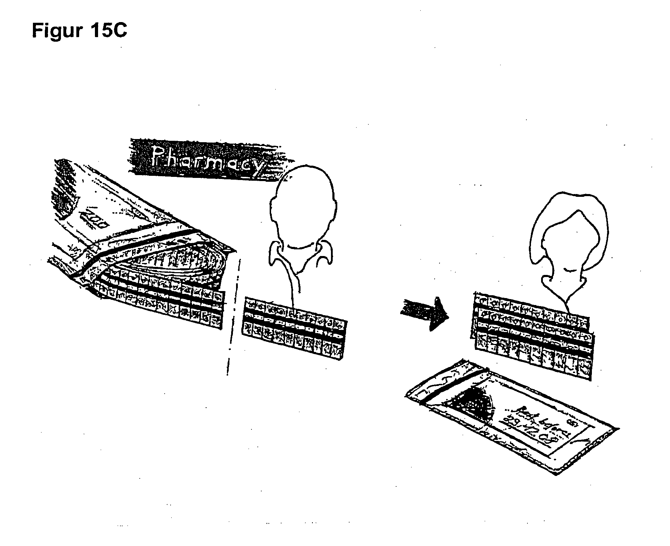

**Figur 15****:** Darstellung eines erfindungsgemäßen Vorratsbehälters in Form einer Aluminiumdose (Figur 15A), eines Tic Tac^{®}-Spenders (Figur 15B) bzw. eines Kunststoffbeutels (Figur 15C), welche jeweils eine Vielzahl miteinander verbundener, einzeln abtrennbarer Teststreifen in rollenförmiger Anordnung enthalten.

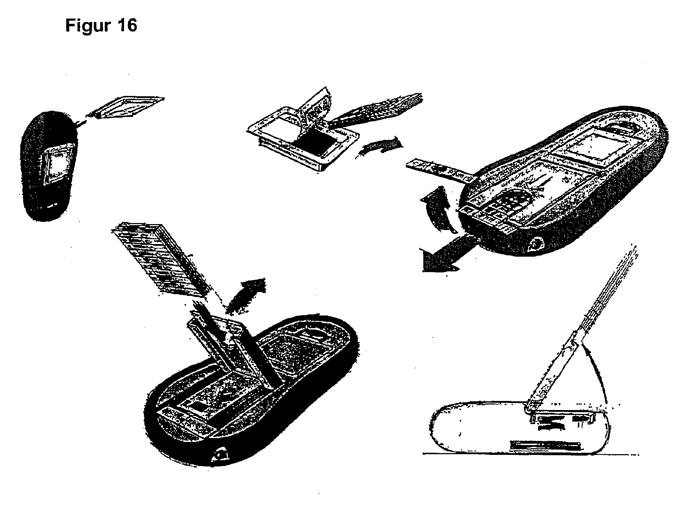

**Figur 16****:** Darstellung eines erfindungsgemäßen analytischen Messgeräts mit integriertem Vorratsbehälter, welcher eine Vielzahl miteinander verbundener, einzeln abtrennbarer Teststreifen in riegelförmiger Anordnung enthält.

### Beispiel 1

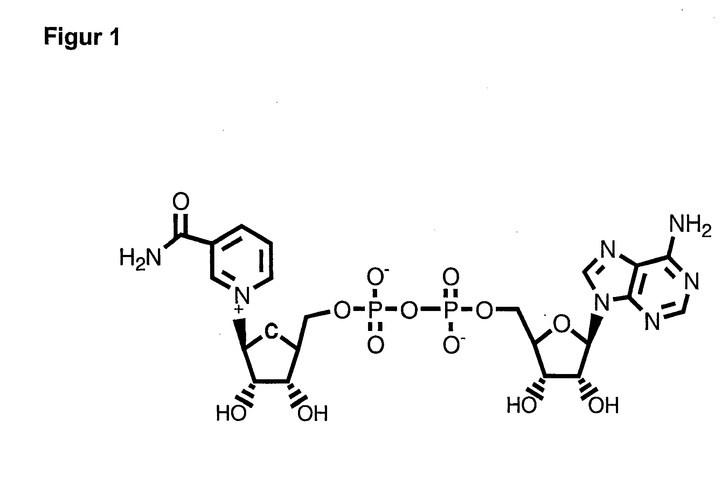

Der Glucose-spezifischen GlucDH wurden carbaNAD (Fig. 1) oder NAD zugesetzt. Diese Rezepturen wurden jeweils auf Pokalonfolie (Lonza) aufgetragen und nach Trocknung unter warmen und feuchten Bedingungen (32°C, 85% relative Luftfeuchtigkeit) eingelagert. Anschließend wurden in regelmäßigen Abständen die Reaktionskinetik sowie die Funktionskurve bestimmt. Parallel wurde zu den jeweiligen Messterminen eine cNAD/NAD-Analytik sowie eine Bestimmung der Restaktivität des Enzyms vorgenommen.

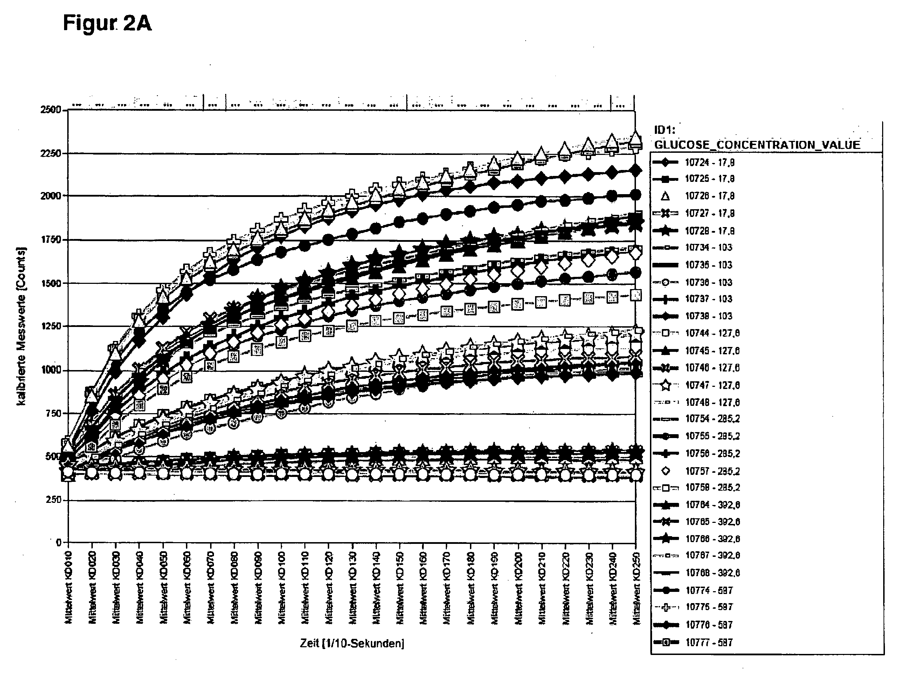

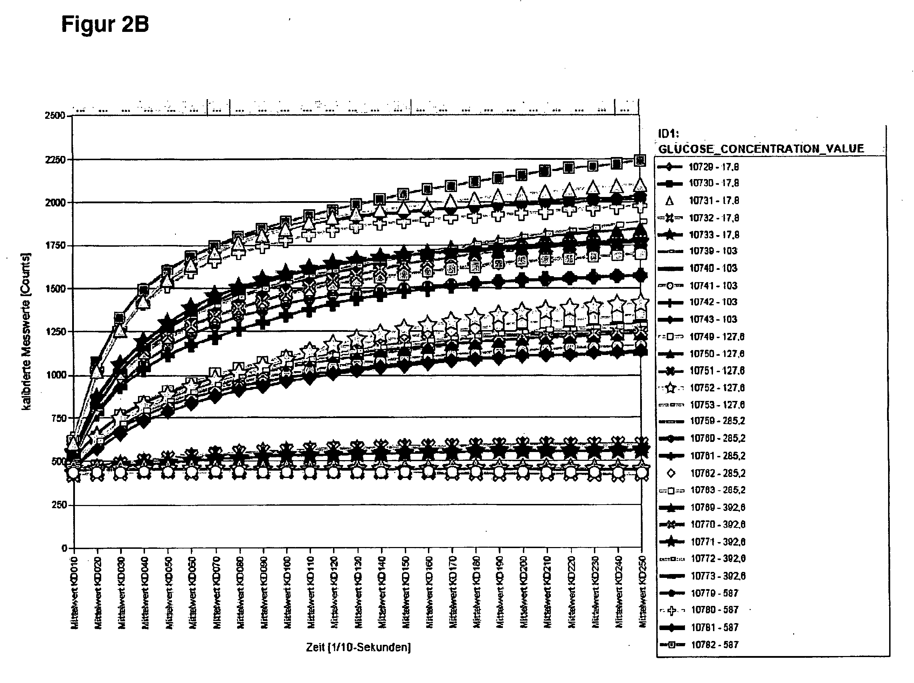

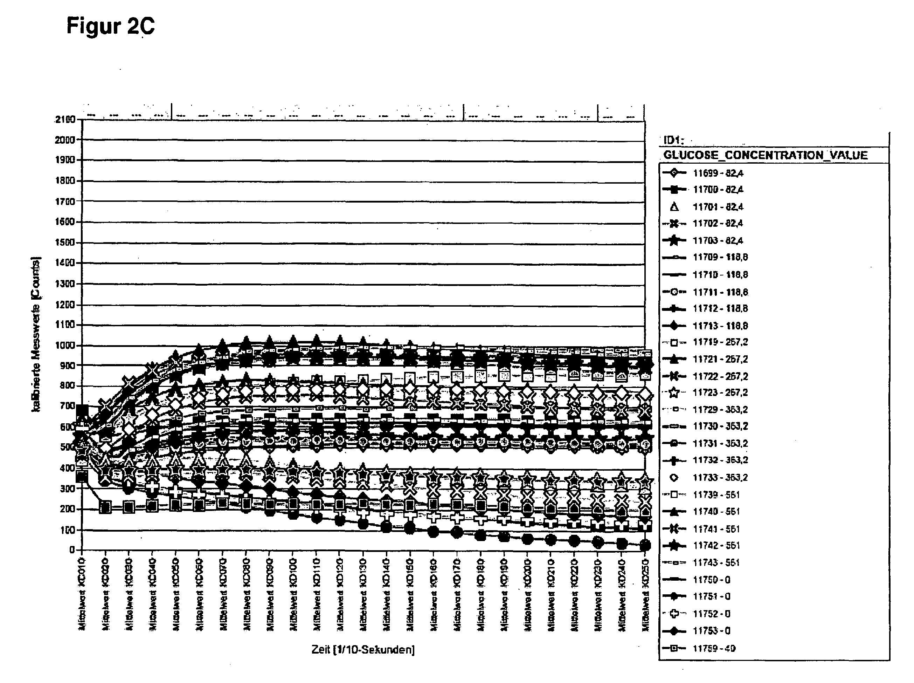

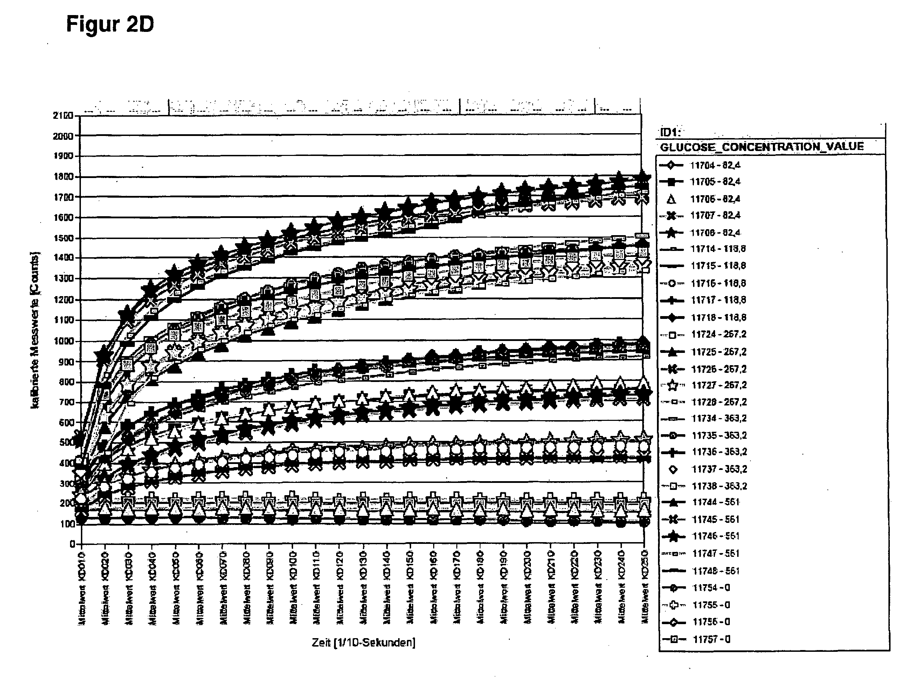

Die am ersten Tag bestimmten Kinetik-Kurven für NAD (Figur 2A) und cNAD (Figur 2B) sind vergleichbar und zeigen auch einen ähnlichen Hub in der Glucoseabhängigkeit. Nach 5 Wochen ist jedoch ein deutlicher Unterschied in den Kinetik-Kurven zu erkennen. Während die Kinetik für NAD (Figur 2C) stark in ihrer Dynamik nachlässt, bleibt die Kinetik des mit cNAD stabilisierten Enzyms praktisch unverändert (Figur 2D).

Auch in den Blankwerten (Trockenleerwert vor Auftrag einer Blutprobe) zeigt sich ein deutlicher Unterschied, wie aus Figur 3 ersichtlich ist. Der Anstieg des Trockenleerwerts bei NAD ist auf die Bildung von fluoreszierenden Teilchen zurückzuführen (N.J. Oppenheimer, in "The Pyridine Nucleotide Coenzyms", Academic Press New York, London 1982, Hrsg. J. Everese, B. Anderson, K. You, Kapitel 3, Seiten 56-65). Überraschenderweise geschieht dies nicht bei cNAD.

Die unterschiedliche Stabilität von Glucose-Dehydrogenase in Gegenwart von NAD bzw. cNAD ist auch aus einem Vergleich der Figuren 4 und 5 ersichtlich. Nach 5 Wochen liegt die Funktionskurve bei dem mit cNAD stabilisierten Enzym noch in der Schar der voherigen Messungen (Figur 5A), während bei dem mit NAD versetzten Enzym (Figur 4) die Kurve bei höheren Konzentrationen abknickt, was ein typisches Zeichen für zu geringe Mengen an Enzym/Coenzym ist. Figur 5B zeigt verschiedene Funktionskurven der mit cNAD stabilisierten Glucose-Dehydrogenase über einen Zeitraum von 24 Wochen. In diesem Zusammenhang wird deutlich, dass sich die Funktion des Enzyms bei hohen Glucose-Konzentrationen über den gesamten Zeitraum hinweg nur geringfügig verändert und nach 24 Wochen etwa dem nach 5 Wochen erhaltenen Wert entspricht.

Die Beziehung zwischen Struktur des Coenzyms und dessen Stabilität über einen vorbestimmten Zeitraum ergibt sich aus Figur 6. Demnach beträgt der Restgehalt an cNAD in einem Glucose-Nachweisreagenz nach 24 Wochen Lagerung (bei 32°C und 85% relativer Luftfeuchtigkeit) noch etwa 80% des Ausgangswertes, während sich der Gehalt an NAD in einem mit NAD stabilisierten Glucose-Nachweisreagenz bereits nach 5 Wochen auf etwa 35% des Ausgangswertes verringert und gemäß Extrapolation nach etwa 17 Wochen auf null reduziert.

Vollkommen überraschend ist das Ergebnis der Restaktivitätsbestimmung des aktiven Enzyms GlucDH nach 5 Wochen bei 32°C und 85% relative Luftfeuchtigkeit (Figur 7A). Das mit NAD stabilisierte Enzym weist nur noch eine extrem geringe Enzymaktivität auf (0,5%), während das mit cNAD stabilisierte Enzym noch eine Restaktivität von 70% besitzt (jeweils im Vergleich zu den mit Trockenmittel (TM) im Kühlschrank (KS) eingelagerten Proben). Nach 24 Wochen bei 32°C und 85% relativer Luftfeuchtigkeit (Figur 7B) beträgt die Restaktivität des Enzyms bei Stabilisierung mit cNAD noch immer etwa 25%.

Auch eine Lagerung von Glucose-Dehydrogenase in flüssiger Phase zeigt deutlich den Unterschied zwischen NAD bzw. cNAD (Figuren 8A und 8B). Nach 95 Stunden bei 50°C liegt die Restaktivität von Glucose-Dehydrogenase in Gegenwart des nativen Coenzyms NAD bei » 5%, während die Restaktivität der GlucDH in Gegenwart des artifiziellen Coenzyms cNAD bei 75% liegt (Figur 8A). Nach 336 Stunden Lagerung bei 50°C beträgt die Restaktivität des mit NAD stabilisierten Enzyms nur mehr etwa 1%; für das in Gegenwart von cNAD gelagerte Enzym wird eine Restaktivität von immer noch etwa 70% beobachtet. Die entsprechenden SDS-Gele zeigen ebenfalls eine Veränderung der GlucDH-Bande in Gegenwart des nativen Coenzyms NAD: es zeigen sich neue Banden bei höheren Molmassen und eine Verschiebung der 30 kDa-Bande.

Insgesamt ist es ein höchst überraschendes Ergebnis, dass die Stabilisierung des Cofaktors gleichzeitig eine Stabilisierung des Enzyms bewirkt - und dies nicht allein durch den kooperativen Effekt des besseren Zusammenhaltes des Enzyms. Der Zerfall des Cofaktors NAD hat einen negativen Effekt auf die Stabilität des Enzyms GlucDH und beschleunigt sogar dessen Inaktivierung. Der Austausch von nativem NAD durch künstliche Analoga erlaubt eine Lagerung der GlucDH unter Stressbedingungen (z.B. erhöhte Temperatur) auch in Gegenwart eines Cofaktors.

Mit einem solchen System lassen sich Blutglucose-Teststreifen mit erheblich verbesserten Stabilitätseigenschaften generieren, bei denen eine Darreichungsform ohne Trockenmittel möglich ist.

### Beispiel 2

Einer Alkohol-Dehydrogenase umfassenden Nachweislösung wurden cNAD oder NAD zugesetzt. Diese Mischungen wurden bei 35°C eingelagert. Anschließend wurde in regelmäßigen Abständen die Stabilität des Enzyms überprüft und eine Bestimmung der Restaktivität des Enzyms vorgenommen.

Wiederum zeigt eine Lagerung in flüssiger Phase den Unterschied zwischen einer Lagerung in Anwesenheit von NAD bzw. cNAD (Figur 9). Nach 65 Stunden bei 35°C liegt die Restaktivität von Alkohol-Dehydrogenase in Gegenwart des nativen Coenzyms NAD bei etwa 6%, während die Restaktivität des Enzyms in Gegenwart des artifiziellen Coenzyms cNAD noch bei etwa 60% liegt.

### Beispiel 3

Figur 10A zeigt im Querschnitt exemplarisch eine Ausführungsform eines Vorratsbehälters der vorliegenden Erfindung in Form eines Bandmagazins, welches in ein analytisches Messgerät eingesetzt werden kann. Der Vorratsbehälter (90) weist ein Gehäuse (91) auf, in welchem eine Vorratsspule (100) und eine Abzugspule (101) positioniert sind. Der Vorratsbehälter umfasst ferner eine Umlenkspitze (20), über deren äußeres Ende ein Testband (30) läuft. Am inneren Ende der Umlenkspitze weist das Bandmagazin eine erste Aussparung (93) zur Aufnahme optischer Mittel auf.

In der dargestellten Ausführungsform weist das Bandmagazin darüber hinaus Rollen oder Stifte (92) zum Hindurchführen des Testbandes durch das Bandmagazin sowie eine zweite Aussparung (94) zur Aufnahme eines Spulenantriebs auf. Die Abzugspule mit Rotationsachse (95) weist hierzu eine Aussparung in ihrer Mitte sowie Eingriffelemente zur Aufnahme des Spulenantrieb auf.

Figur 10B zeigt im Querschnitt ein analytisches Messgerät (10), in welches das in Figur 10A beschriebene Bandmagazin (90) eingesetzt ist. Das analytische Messgerät weist einen Spulenantrieb (102) auf, welcher in die Abzugspule (101) des Bandmagazins eingreift und beispielsweise über einen Motor angetrieben werden kann. Der Spulenantrieb ermöglicht hierbei eine Steuerung und Koordinierung des Transports von Testband (30) in eine Probenaufgabeposition bzw. Messposition an der Umlenkspitze (20) des Bandmagazins, wobei die Messung des mit einer Probe des Analyten benetzten Testbandes gegebenenfalls auch in einer anderen Position, wie beispielsweise im Inneren des Bandmagazins, erfolgen kann.

Das analytische Messgerät umfasst ferner optische Mittel (99), welche in die erste Aussparung (93) aufgenommen werden, sobald das Bandmagazin in das analytische Messgerät eingebracht wird. Sofern eine optische Messung des Analyten vorgesehen ist, so muss die Optik des analytischen Messgeräts mit der Umlenkspitze (20) gekoppelt werden, was beispielsweise durch Verwendung einer optisch transparenten Umlenkspitze oder durch Einbringung optischer Fasern in eine ansonsten lichtundurchlässige Umlenkspitze bewerkstelligt werden kann. Die Optik des analytischen Messgeräts kann beispielsweise die Verwendung optischer Fasern vorsehen, an welche eine Lichtquelle und ein Detektor gekoppelt sind.

Figur 10B zeigt weiterhin einen Kanal (96) innerhalb des Bandmagazins, welcher zwischen der Umlenkspitze und der Vorratsspule positioniert ist und in welchem das Testband vom Vorratsbereich (97) des Bandmagazins zur Umlenkspitze (20) geführt wird, bevor es nach Inkontaktbringen mit einer Probe des Analyten an der Umlenkspitze in den Abzugbereich (98) des Bandmagazins zurückgeführt wird. Hierbei kann der Abstand einzelner Testbereiche des Testbandes beipielsweise derart gewählt werden, dass ein zweiter Testbereich (30") noch innerhalb des Bandmagazins positioniert ist, während ein erster Testbereich (30') sich in der Probenaufgabeposition bzw. Messposition an der Umlenkspitze (20) befindet, um gegbenenfalls einen Schutz gegenüber Feuchtigkeit oder anderen äußeren Einflüssen zu gewährleisten.

### Beispiel 4

Figur 11 zeigt im Querschnitt exemplarisch eine Ausführungsform eines Vorratsbehälters der vorliegenden Erfindung in Form eines scheibenförmigen Wendemagazins, welches in ein analytisches Messgerät (114) eingesetzt werden kann. Das analytische Messgerät (114) weist dabei ein Gehäuse (112) mit einer Fingeröffnung (116) auf, durch welche eine Probennahmebewegung erfolgen kann.

Der Vorratsbehälter (110) weist zwei Teilmagazine in Form von Magazinhälften (172, 174) auf, welche im Wesentlichen symmetrisch zueinander ausgestaltet sind. Im Einzelnen umfasst der Vorratsbehälter (110) ein Gehäuse (122) mit einer runden Mittelöffnung (124) sowie zwei Hilfsmittelebenen (118, 120), welche jeweils parallel übereinander angeordnet sind und parallel zur Ebene des Vorratsbehälters verlaufen. In dem Gehäuse (122) sind in jeder der beiden Hilfsmittelebenen (118, 120) eine Vielzahl von Kammern (126) aufgenommen, wobei die Kammern (126) der zweiten Hilfsmittelebene (120) jeweils um eine halbe Einheit verdreht unterhalb der Kammern (126) der ersten Hilfsmittelebene (118) liegen. Der Vorratsbehälter (110) kann somit aus zwei identischen Halbebenen zusammengesetzt sein, welche gegeneinander montiert sind und jeweils um eine halbe Einheit, d.h. um einen halben Winkelabstand der Kammer (126), gegeneinander verdreht sind.

In den Kammern (126) sind jeweils analytische Hilfsmittel (128) aufgenommen, welche ihrerseits aus mehreren Teilhilfsmitteln (130) zusammengesetzt sind. Ein erstes Teilhilfsmittel (130) umfasst einen in den Kammern (126) radial gelagerten Microsampler (132), welcher seinerseits eine Lanzette (134) und ein von den Lanzetten (134) radial nach innen verlaufendes Kapillarelement (136) umfasst. An ihrem der Mittelöffnung (124) zuweisenden Ende weisen die Microsampier (132) jeweils Kopplungselemente (138) auf, welche in Form von Ösen ausgestaltet sein können, in die ein (in Figur 11 nicht dargestellter) Aktor des analytischen Messgeräts (114) über einen hakenförmigen Stößel einer Aktorstange von der Mittelöffnung (124) her eingreifen kann. Als zweites Teilhilfsmittel (130) weisen die analytischen Hilfsmittel (128) ein diagnostisches Testelement (140) auf, welches eine Testchemie (142) in Form eines Testbereichs (144) umfasst und in das Gehäuse (122) integriert sein kann.

Der Vorratsbehälter (110) ist derart ausgestaltet, dass die analytischen Hilfsmittel (128) in den beiden Hilfsmittelebenen (118, 120) versetzt zueinander angeordnet sind. Dementsprechend liegt jeweils ein Testelementfenster (168) einer der beiden Hilfsmittelebenen (118, 120) zwischen zwei Kammern (126) der jeweils anderen Hilfsmittelebene (118, 120). Auf diese Weise ist sichergestellt, dass mittels des analytischen Messgeräts jeweils, ungestört von den benachbarten Kammern (126) der jeweils anderen Hilfsmittelebene (118, 120), die Rückseite des Testbereichs (144) in seiner Applikationsposition beobachtet werden kann. Die versetzte Anordnung der Kammern (126) erlaubt also einen beiderseitigen Zugriff auf die jeweils aktiven diagnostischen Testelemente (140) in ihrer Applikationsposition. Die restlichen analytischen Hilfsmittel (128) im Vorratsbehälter (110) werden dabei im Allgemeinen nicht freigelegt und bleiben durch ihre entsprechenden Versiegelungen bis zur Verwendung steril.

## Patentansprüche

1. Vorratsbehälter, enthaltend mindestens ein diagnostisches Testelement, welches ein Enzym und ein stabilisiertes Coenzym umfasst,
**dadurch gekennzeichnet,**
**dass** er
(a) im wesentlichen frei von Trocknungsmitteln ist und
(b) keine Dichtungselemente umfasst, welche das Eindringen von Feuchtigkeit aus der Umgebung in den Vorratsbehälter im wesentlichen verhindern.

2. Vorratsbehälter, enthaltend mindestens ein diagnostisches Testelement, welches ein Enzym und ein stabilisiertes Coenzym umfasst,
**dadurch gekennzeichnet,**
**dass** er einen Feuchtigkeitsaustausch zwischen unverbrauchten Testbereichen und verbrauchten Testbereichen des Testelements oder der Testelemente, ermöglicht, wobei die unverbrauchten Testbereiche und die verbrauchten Testbereiche in einer einzigen Kammer oder in zwei durch eine feuchtigkeitsdurchlässige Trennwand voneinander getrennten Kammern gelagert sind.

3. Vorratsbehälter nach Anspruch 1 oder 2,
**dadurch gekennzeichnet,**
**dass** das Testelement einen oder mehrere Testbereiche umfasst, wobei jeweils ein einzelner Testbereich des Testelements mit einem analytischen Messgerät wechselwirken kann.

4. Vorratsbehälter nach einem der Ansprüche 1 bis 3,
**dadurch gekennzeichnet,**
**dass** er eine Probenaufgabeposition, eine Messposition, und mindestens eine Lagerungsposition für das Testelement umfasst.

5. Vorratsbehälter nach einem der Ansprüche 1 bis 4,
**dadurch gekennzeichnet,**
**dass** er zumindest teilweise aus mindestens einem wasserdampfdurchlässigen Material, insbesondere mindestens einem Kunststoff ausgewählt aus der Gruppe bestehend aus Polyamid, Polycarbonat, Polyester, Polyethylen und Polypropylen, besteht, oder/und ausschließlich Eintrittsöffnungen mit einem Durchmesser von ≤ 100 µm, insbesondere von ≤ 20 µm, aufweist.

6. Vorratsbehälter nach einem der Ansprüche 1 bis 5,
**dadurch gekennzeichnet,**
**dass** es sich um ein Bandmagazin oder ein Teststreifenmagazin, insbesondere ein Blistermagazin, Leporellomagazin, Scheibenmagazin, Stapelmagazin, Trommelmagazin oder Wendemagazin, handelt, wobei das Teststreifenmagazin bevorzugt eine Vielzahl miteinander verbundener, einzeln abtrennbarer Teststreifen in riegelförmiger oder/und rollenförmiger Anordnung umfasst.

7. Vorratsbehälter nach einem der Ansprüche 1 bis 6,
**dadurch gekennzeichnet,**
**dass** das Enzym eine Nikotinamid-Adenin-Dinukleotid (NAD/NADH)- oder Nikotinamid-Adenin-Dinukleotidphosphat (NADP/NADPH)-abhängige Dehydrogenase, insbesondere eine Glucose-Dehydrogenase (EC 1.1.1.47) oder eine Glucose-6-phosphat-Dehydrogenase (EC 1.1.1.49), ist.

8. Vorratsbehälter nach einem der Ansprüche 1 bis 7,
**dadurch gekennzeichnet,**
**dass** das stabilisierte Coenzym eine stabilisierte NAD(P)/NAD(P)H-Verbindung, insbesondere carbaNAD, ist.

9. Vorratsbehälter nach einem der Ansprüche 1 bis 8,
**dadurch gekennzeichnet,**
**dass** das Testelement weiterhin einen Mediator, insbesondere einen Mediator ausgewählt aus der Gruppe bestehend aus einem 1,10-Phenanthrolinchinon, einem 1,7-Phenanthrolinchinon und einem 4,7-Phenanthrolinchinon, oder/und ein Nadelelement zum Anritzen von Haut umfasst.

10. Vorratsbehälter nach einem der Ansprüche 1 bis 9,
**dadurch gekennzeichnet,**
**dass** das Testelement im wesentlichen frei von Trocknungsmitteln ist.

11. Vorratsbehälter nach einem der Ansprüche 1 bis 10,
**dadurch gekennzeichnet,**
**dass** es sich bei dem Testelement um ein Testband, insbesondere um ein durchgängiges Testband, eine Testdisc, ein Testpad oder einen Teststreifen handelt.

12. Vorratsbehälter nach einem der Ansprüche 1 bis 11,
**dadurch gekennzeichnet,**
**dass** das Testelement nach Lagerung in dem Vorratsbehälter für mindestens 4 Wochen, bevorzugt mindestens 8 Wochen, besonders bevorzugt mindestens 12 Wochen bei einer Temperatur von mindestens 20°C, bevorzugt mindestens 25 °C, besonders bevorzugt mindestens 30 °C eine Abnahme der Enzymaktivität von weniger als 50 %, bevorzugt von weniger als 30 %, besonders bevorzugt von weniger als 20 % gegenüber dem Ausgangswert der Enzymaktivität aufweist.

13. Analytisches Messgerät, umfassend einen Vorratsbehälter nach einem der Ansprüche 1 bis 12.

## Claims

1. Container, containing at least one diagnostic test element, which comprises an enzyme and a stabilised coenzyme,
**characterised in that**
it
(a) is essentially free from drying agents and
(b) comprises no sealing elements, which essentially prevent moisture from getting into the container from the surroundings.

2. Container, containing at least one diagnostic test element, which comprises an enzyme and a stabilised coenzyme,
**characterised in that**
it makes an exchange of moisture possible between unused test areas and used test areas of the test element or test elements, in which the unused test areas and the used test areas are stored in a single chamber or in two chambers separated from each other by a partition that is permeable to moisture.

3. Container according to claim 1 or 2,
**characterised in that**
the test element comprises one or several test areas, in which an individual test area of the test element may interact with an analytical measuring device.

4. Container according to one of claims 1 to 3,
**characterised in that**
it has a sampling position, a measuring position and at least one storing position for the test element.

5. Container according to one of claims 1 to 4,
**characterised in that**
it consists at least partly of at least one material that is permeable to steam, particularly at least one plastic selected from the group consisting of polyamide, polycarbonate, polyester, polyethylene and polypropylene, and/or has inlet openings exclusively with a diameter of ≤ 100 µm, particularly ≤ 20 µm.

6. Container according to one of claims 1 to 5,
**characterised in that**
it is a band cartridge or test strip cartridge, particularly a blister cartridge, concertina cartridge, disc cartridge, stacking cartridge, drum cartridge or turning cartridge, in which the test trip cartridge comprises preferably a multitude of test strips connected to each other, which may be separated individually, in a bar type and/or a roll type arrangement.

7. Container according to one of claims 1 to 6,
**characterised in that**
the enzyme is a nicotinamide adenine dinucleotide (NAD/NADH) or nicotinamide adenine dinucleotide phosphate (NADP/NADPH) dependent dehydrogenase, particularly a glucose dehydrogenase (EC 1.1.1.47) or a glucose-6-phosphate dehydrogenase (EC 1.1.1.49).

8. Container according to one of claims 1 to 7,
**characterised in that**
the stabilised coenzyme is a stabilised NAD/(P)/NAD(P)H compound, particularly carbaNAD.

9. Container according to one of claims 1 to 8,
**characterised in that**
the test element also comprises a mediator, particularly a mediator selected from the group consisting of a 1,10-phenanthrolinchinon, a 1,7-phenanthrolinchinon and a 4,7-phenanthrolinchinon, and/or a needle element for scratching skin.

10. Container according to one of claims 1 to 9,
**characterised in that**
the test element is essentially free for drying agents.

11. Container according to one of claims 1 to 10,
**characterised in that**
the test element is a test band, particularly a continuous test band, a test disc, a test pad or a test strip.

12. Container according to one of claims 1 to 11,
**characterised in that**
after storing in the container for at least 4 weeks, preferably at least 8 weeks, particularly preferably at least 12 weeks, at a temperature of at least 20° C, preferably at least 25° C, particularly preferably at least 30° C, the test element shows a decrease in enzyme activity of less than 50%, preferably less the 30%, particularly preferably less than 20%, compared with the initial value for enzyme activity.

13. Analytical measuring device, comprising a container according to one of claims 1 to 12.

## Revendications

1. Récipient de stockage contenant au moins un élément de test de diagnostic, qui comprend une enzyme et une coenzyme stabilisée, **caractérisé en ce qu'il**
(a) est sensiblement exempt d'agent de séchage et
(b) ne comprend pas d'élément d'étanchéité qui empêchent essentiellement la pénétration de l'humidité de l'air ambiant dans le récipient de stockage.

2. Récipient de stockage comprenant au moins un élément de test de diagnostic, qui comprend une enzyme et une coenzyme stabilisée,
**caractérisé en ce qu'il**
permet un échange d'humidité entre les domaines de test non utilisés et les domaines de test utilisés de l'élément de test ou des éléments de test, dans lequel les domaines de test non utilisés et les domaines de test utilisés sont positionnés dans une seule chambre ou dans deux chambres séparées l'une de l'autre par une paroi de séparation perméable à l'humidité.

3. Récipient de stockage selon la revendication 1 ou 2,
**caractérisé en ce que**
l'élément de test comprend un ou plusieurs domaines de test, dans lequel chaque domaine individuel de test de l'élément de test peut intéragir avec un appareil de mesure d'analyses.

4. Récipient de stockage selon l'une quelconque des revendications 1 à 3,
**caractérisé en ce qu'il**
comprend un emplacement pour l'ajout de l'échantillon, un emplacement de mesure, et au moins un emplacement de stockage pour l'élément de test.

5. Récipient de stockage selon l'une quelconque des revendications 1 à 4,
**caractérisé en ce qu'il**
se compose au moins en partie d'un matériel perméable à la vapeur d'eau, en particulier au moins d'une matière plastique choisie parmi le groupe constitué du polyamide, polycarbonate, polyester, polyéthylène et polypropylène, et/ou présente exclusivement des ouvertures d'entrée ayant un diamètre de ≤ 100 µm, en particulier de ≤ 20 µm.

6. Récipient de stockage selon l'une quelconque des revendications 1 à 5,
**caractérisé en ce qu'il**
s'agit d'un magasin de bandes, d'un magasin de bandelettes de test, en particulier un magasin de blister, un magasin de papier de pliage, un magasin de lamelles, un magasin à piles, un magasin à tambour ou un magasin à tournant, dans lequel le magasin de bandelettes de test comprend de préférence une pluralité de bandelettes de test liées les unes aux autres, séparables individuellement disposées en forme de barre ou/et en forme de rouleau.

7. Récipient de stockage selon l'une quelconque des revendications 1 à 6,
**caractérisé en ce que**
l'enzyme est une nicotinamide-adénine-dinucléotide (NAD/NADH) ou déshydrogénase dépendante du nicotinamide-adénine-dinucléotide-phosphate (NADP/NADPH), en particulier une glucose déshydrogénase (EC 1.1.1.47) ou une glucose-6-phosphate déshydrogénase (EC 1.1.1.49).

8. Récipient de stockage selon l'une quelconque des revendications 1 à 7,
**caractérisé en ce que**
la coenzyme stabilisée est un composé stabilisé de NAD(P)/NAD(P)H, en particulier carbaNAD.

9. Récipient de stockage selon l'une quelconque des revendications 1 à 8,
**caractérisé en ce que**
l'élément de test comprend de plus un médiateur, en particulier un médiateur choisi dans le groupe consistant en une quinone 1,10-phénanthroline, une quinone 1,7-phénanthroline et une quinone 4,7-phénanthroline, ou/et un élément d'aiguille pour tracer la peau.

10. Récipient de stockage selon l'une quelconque des revendications 1 à 9, **caractérisé en ce que**
l'élément de test est essentiellement exempt d'agent de séchage.

11. Récipient de stockage selon l'une quelconque des revendications 1 à 10,
**caractérisé en ce que**
l'élément d'essai est une bande d'essai, en particulier à une bande continue de test, un disque de test, un tampon d'essai ou une bandelette de test.

12. Récipient de stockage selon l'une quelconque des revendications 1 à 11,
**caractérisé en ce que**
l'élément de test après stockage dans le récipient de stockage pendant au moins 4 semaines, de préférence au moins 8 semaines, plus préférablement au moins 12 semaines à une température d'au moins 20°C, de préférence d'au moins 25 °C, plus préférablement d'au moins 30 °C présente une diminution de l'activité enzymatique inférieur à 50%, de préférence inférieure à 30%, plus préférablement inférieur à 20% par rapport à la valeur de départ de l'activité enzymatique.

13. Appareil de mesure d'analyses, comprenant un récipient de stockage selon l'une quelconque des revendications 1 à 12.
